# EUROPEAN PATENT APPLICATION

(11) **EP 2 407 465 A1**
(43) Date of publication of application: **18.01.2012**
(21) Application number: 10750884.8
(22) Date of filing: 10.03.2010
(51) Int. Cl.: C07D 333/78, H01L 29/786, H01L 29/80, H01L 51/05, H01L 51/30, H01L 51/42

(54) **CONJUGATED COMPOUND, AND ORGANIC THIN FILM AND ORGANIC THIN FILM ELEMENT EACH COMPRISING SAME**

(30) Priority: 11.03.2009 JP 2009058737
(71) Applicant: Sumitomo Chemical Company, Limited, Tokyo 104-8260 (JP); Osaka University, Suita-shi Osaka 565-0871 (JP)
(72) Inventor: IE, Yutaka, Suita-shi Osaka 565-0871 (JP); OKABE, Makoto, Suita-shi Osaka 565-0871 (JP); ASO, Yoshio, Suita-shi Osaka 565-0871 (JP); UEDA, Masato, Tsukuba-shi Ibaraki 305-0046 (JP)
(74) Representative: Duckworth, Timothy John
(86) International application number: PCT/JP2010/054050
(87) International publication number: WO 2010/104131

(57) **Abstract**

A conjugated compound having a group represented by formula (I) and/or formula (II). 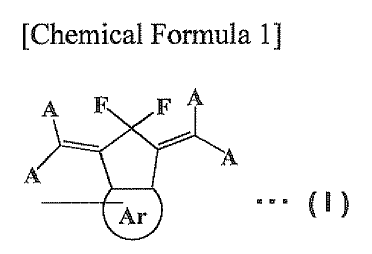 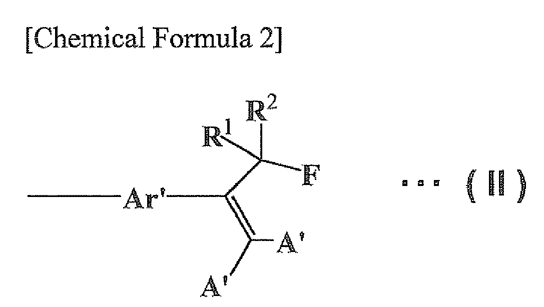 [In the formulas, Ar represents an optionally substituted trivalent aromatic hydrocarbon or optionally substituted trivalent heterocyclic group, and A represents hydrogen, a halogen atom or a monovalent group. When multiple A groups are present they may be the same or different, and at least one A represents an electron-withdrawing group. Ar' represents an optionally substituted C6 or greater divalent aromatic hydrocarbon or optionally substituted C4 or greater divalent heterocyclic group, and R¹ and R² are the same or different and each represents hydrogen, a halogen atom or a monovalent group, while A' represents hydrogen, a halogen atom or a monovalent group. When multiple A' groups are present they may be the same or different, and at least one A' represents an electron-withdrawing group.]

## Description

### Technical Field

The present invention relates to a conjugated compound, and to an organic thin-film and an organic thin-film element comprising it.

### Background Art

A variety of conjugated compounds have been developed as organic n-type semiconductors, for use as materials in organic thin-film elements such as organic transistors, organic solar cells and optical sensors. Specific ones that have been proposed include compounds having fluoroalkyl, groups introduced at the ends of oligothiophenes (Patent document 1).

### Citation List

### Patent Literature

[Patent document 1] International Patent Publication No. WO2003/010778

### Summary of Invention

### Technical Problem

The compounds mentioned above, however, cannot be utilized as organic n-type semiconductors with satisfactory electron transport properties.

It is therefore an object of the present invention to provide novel conjugated compounds that can be used as organic n-type semiconductors with excellent electron transport properties. It is another object of the invention to provide organic thin-films containing the novel conjugated compounds and organic thin-film elements comprising the organic thin-films.

### Solution to Problem

In order to achieve the object stated above, the invention provides a conjugated compound having a group represented by formula (I) and/or a group represented by formula (II).

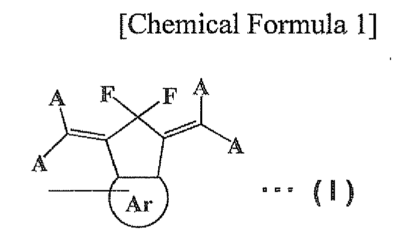

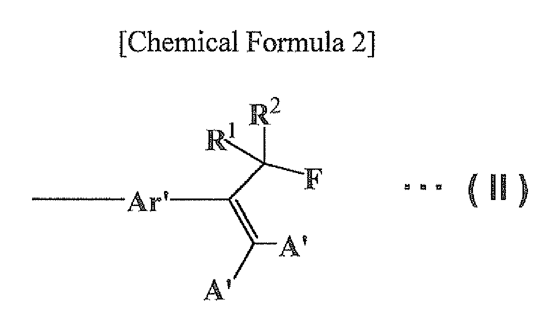

In the formulas, Ar represents an optionally substituted trivalent aromatic hydrocarbon or optionally substituted trivalent heterocyclic group. A represents hydrogen, a halogen atom or a monovalent group, and when multiple A groups are present, they may be the same or different, and at least one A is an electron-withdrawing group. Ar' represents an optionally substituted C6 or greater divalent aromatic hydrocarbon or optionally substituted C4 or greater divalent heterocyclic group, and R¹ and R² are the same or different and each represents hydrogen, a halogen atom or a monovalent group. A' represents hydrogen, a halogen atom or a monovalent group, and when multiple A' groups are present, they may be the same or different, and at least one A' is an electron-withdrawing group.

A conjugated compound comprising such a backbone has an excellent intermolecular packing property, and introduction of the structure containing a combination of fluorine and at least one electron-withdrawing group (the structure -C(=CA"₂)-C(F)< (where A" represents A or A' above) allows a sufficiently low LUMO to be exhibited. The conjugated compound is therefore sufficiently suitable as an n-type semiconductor with excellent electron injection and electron transport properties. Such compounds are also chemically stable and have excellent solubility in solvents, and therefore thin-film formation is facilitated, and the conjugated compounds can be used to form organic thin-films so that organic thin-film elements with excellent performance can be produced.

From the viewpoint of allowing the LUMO to be lowered even further, the conjugated compound is preferably a conjugated compound with a group represented by formula (III), and more preferably a conjugated compound with 2 or more of such groups.

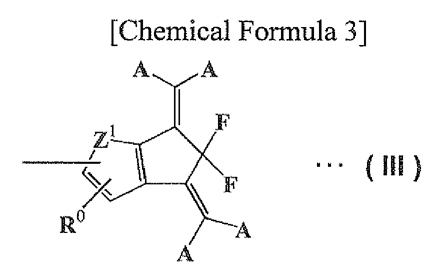

In the formula, A represents hydrogen, a halogen atom or a monovalent group, and when multiple A groups are present, they may be the same or different, and at least one A is an electron-withdrawing group. R° represents hydrogen or a C1-20 alkyl, C1-20 fluoroalkyl, C1-20 alkoxy or C1-20 fluoroalkoxy group. Z¹ represents a group represented by one of formulas (i)-(ix), wherein R³, R⁴, R⁵ and R⁶ are the same or different and each represents hydrogen or a monovalent group, and R³ and R⁴ may be bonded together to form a ring.

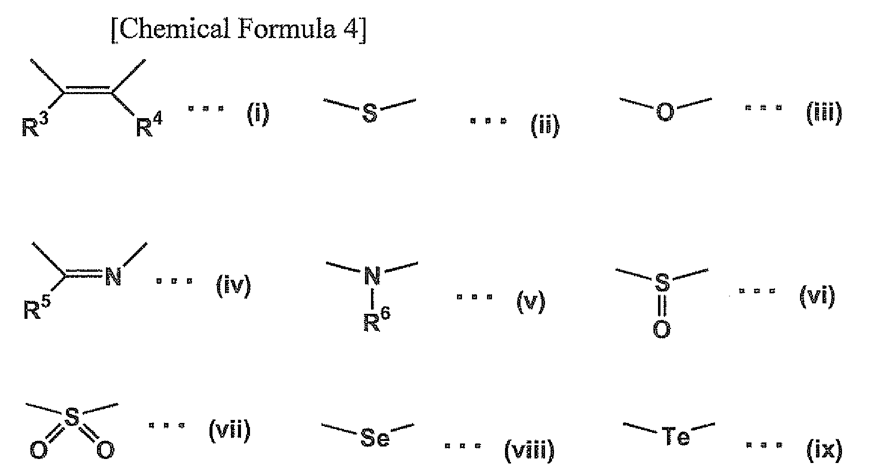

Preferred as such conjugated compounds are conjugated compounds represented by formula (IV). Such conjugated compounds are easy to synthesize, have sufficiently low LUMO and exhibit an excellent electron transport property. They can therefore suitably be used as organic n-type semiconductors.

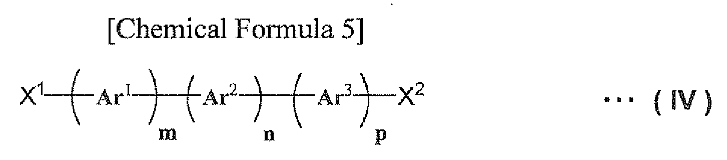

In the formulas, X¹ and X² are the same or different and are each a group represented by formula (III), with X¹ and X² being either the same or different. Ar¹, Ar² and Ar³ are the same or different and each represents an optionally substituted C6 or greater divalent aromatic hydrocarbon or optionally substituted C4 or greater divalent heterocyclic group, and m, n and p are the same or different and each represents an integer of 0-6. This is with the proviso that m+n+p is an integer of 1 or greater.

In the aforementioned conjugated compounds, preferably at least one of Ar¹, Ar² and Ar³ is an optionally substituted thienylene group. In the conjugated compounds mentioned above, Z¹ is preferably a group represented by formula (ii). Also, A is preferably a cyano group. Such groups will allow an even more excellent electron transport property to be obtained.

The conjugated compound is preferably a conjugated compound having a group represented by formula (V) and more preferably a conjugated compound having 2 or more groups represented by formula (V), since this will allow the LUMO to be lowered even further.

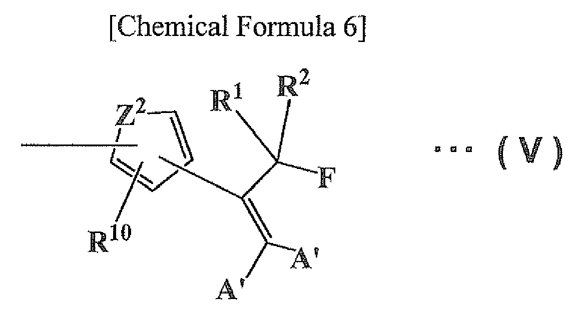

In the formula, R¹ and R² are the same or different and each represents hydrogen, a halogen atom or a monovalent group. A' represents hydrogen, a halogen atom or a monovalent group, and when multiple A' groups are present, they may be the same or different, and at least one A' is an electron-withdrawing group. R¹⁰ represents hydrogen or a C1-20 alkyl, C1-20 fluoroalkyl, C1-20 alkoxy or C1-20 fluoroalkoxy group. Z² represents a group represented by one of formulas (xi)-(xix), wherein R¹³, R¹⁴, R¹⁵ and R¹⁶ are the same or different and each represents hydrogen or a monovalent group, and R¹³ and R¹⁴ may be bonded together to form a ring.

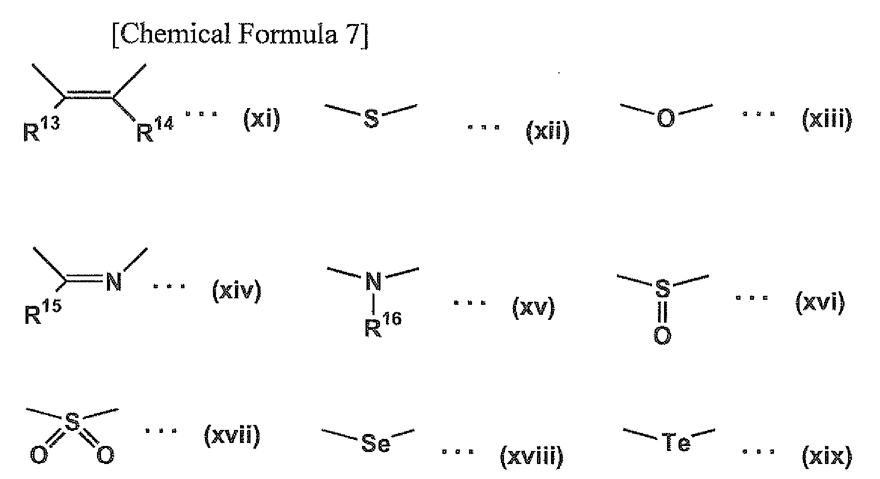

Preferred as such conjugated compounds are conjugated compounds represented by formula (VI). Such conjugated compounds are easy to synthesize, have sufficiently low LUMO and exhibit an excellent electron transport property. They can therefore suitably be used as organic n-type semiconductors.

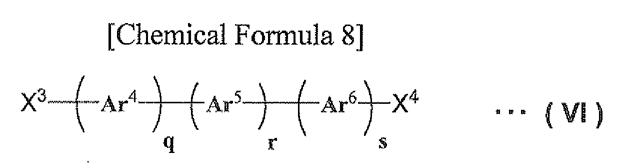

In the formula, X³ and X⁴ are the same or different and each is a group represented by formula (V). Ar⁴, Ar⁵ and Ar⁶ are the same or different and each represents an optionally substituted C6 or greater divalent aromatic hydrocarbon or optionally substituted C4 or greater divalent heterocyclic group, and q, r and s are the same or different and each represents an integer of 0-6. This is with the proviso that q+r+s is an integer of 1 or greater.

In the aforementioned conjugated compounds, preferably at least one of Ar⁴, Ar⁵ and Ar⁶ is an optionally substituted thienylene group. In the conjugated compounds mentioned above, Z² is preferably a group represented by formula (xi) or (xii). Also, A' is preferably a cyano group. Such groups will allow an even more excellent electron transport property to be obtained.

The invention provides organic thin-films comprising the aforementioned conjugated compounds. The invention further provides organic thin-film elements, organic thin-film transistors, organic solar cells and optical sensors comprising the organic thin-films.

Because such organic thin-films, organic thin-film elements, organic thin-film transistors, organic solar cells and optical sensors have sufficiently low LUMO and are formed using conjugated compounds of the invention exhibiting excellent charge transport properties as mentioned above, it is possible to achieve excellent performance.

### Advantageous Effects of Invention

According to the invention it is possible to provide novel conjugated compounds that can be used as organic n-type semiconductors with excellent electron transport properties. It is also possible to provide organic thin-films containing the novel conjugated compounds, and organic thin-film elements comprising the organic thin-films.

### Brief Description of Drawings

Fig. 1 is a schematic cross-sectional view of an organic thin-film transistor according to a first embodiment.
Fig. 2 is a schematic cross-sectional view of an organic thin-film transistor according to a second embodiment.
Fig. 3 is a schematic cross-sectional view of an organic thin-film transistor according to a third embodiment.
Fig. 4 is a schematic cross-sectional view of an organic thin-film transistor according to a fourth embodiment.
Fig. 5 is a schematic cross-sectional view of an organic thin-film transistor according to a fifth embodiment.
Fig. 6 is a schematic cross-sectional view of an organic thin-film transistor according to a sixth embodiment.
Fig. 7 is a schematic cross-sectional view of an organic thin-film transistor according to a seventh embodiment.
Fig. 8 is a schematic cross-sectional view of a solar cell according to an embodiment of the invention.
Fig. 9 is a schematic cross-sectional view of an optical sensor according to a first embodiment.
Fig. 10 is a schematic cross-sectional view of an optical sensor according to a second embodiment.
Fig. 11 is a schematic cross-sectional view of an optical sensor according to a third embodiment.

### Description of Embodiments

Preferred embodiments of the invention will now be explained in detail, with reference to the accompanying drawings as necessary. Throughout the drawings, corresponding elements will be referred to by like reference numerals and will be explained only once. Unless otherwise specified, the vertical and horizontal positional relationships are based on the positional relationships in the drawings. Also, the dimensional proportions depicted in the drawings are not necessarily limitative.

A conjugated compound of the invention has a group represented by formula (I) and/or a group represented by formula (II). A conjugated compound, according to the invention, is a compound comprising a structure with a single bond, and an unsaturated bond, lone electron pair, radical or nonbonded orbital, alternately linked, in the main backbone, with delocalization of electrons due to interaction between π-orbitals or nonbonded orbitals, in part or across the entire main backbone. Preferred are conjugated compounds that are π-conjugated compounds due to interaction between π-orbitals.

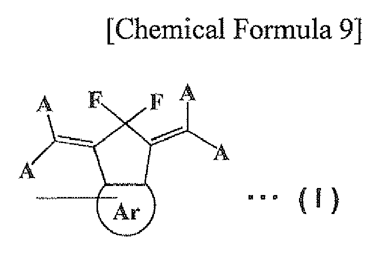

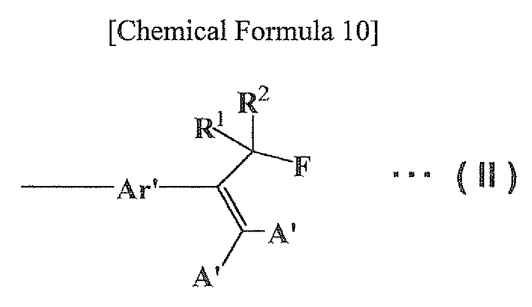

Ar represents an optionally substituted trivalent aromatic hydrocarbon or optionally substituted trivalent heterocyclic group, and A represents hydrogen, a halogen atom or a monovalent group. When multiple A groups are present they may be the same or different, and at least one A represents an electron-withdrawing group. Ar' represents an optionally substituted C6 or greater divalent aromatic hydrocarbon or optionally substituted C4 or greater divalent heterocyclic group, and R¹ and R² are the same or different and each represents hydrogen, a halogen atom or a monovalent group, while A' represents hydrogen, a halogen atom or a monovalent group. When multiple A' groups are present they may be the same or different, and at least one A' represents an electron-withdrawing group.

The conjugated compound may be a compound with a group represented by formula (I), a compound with a group represented by formula (II), or a compound with a group represented by formula (I) and a group represented by formula (II). When the conjugated compound has multiple groups represented by formula (I) or (II) in the molecule, groups represented by the same formulas may be the same or different, but more preferably they are the same from the viewpoint of facilitating synthesis.

In formula (1), A represents hydrogen, a halogen atom or a monovalent group, and when multiple A groups are present, they may be the same or different, and at least one A is an electron-withdrawing group. Preferably, at least two A groups are electron-withdrawing groups and more preferably all of the A groups are electron-withdrawing groups, since this will allow the LUMO to be further lowered. Examples of electron-withdrawing groups include cyano, nitro, aldehyde, acyl, alkoxycarbonyl, carboxyl, hydroxyl and halogen atoms, with cyano, nitro and halogen atoms being preferred, and cyano groups being more preferred. Most preferably, the group represented by formula (I) is one in which all of the A groups are cyano groups.

Monovalent groups for A include groups comprising straight-chain or branched low molecular chains, C3-60 monovalent cyclic groups (monocyclic or fused rings, carbon rings or heterocyclic rings, saturated or unsaturated and optionally substituted), saturated or unsaturated hydrocarbon groups, alkyl groups substituted with hydroxyl, alkoxy, alkanoyloxy, amino, oxyamino, alkylamino, dialkylamino, alkanoylamino, cyano, nitro, sulfo or halogen atoms, alkoxysulfonyl groups (wherein the hydrogens of the alkoxy groups may be substituted with halogen atoms), alkylsulfonyl groups (wherein the hydrogens of the alkyl groups may be substituted with halogen atoms), sulfamoyl, alkylsulfamoyl, carboxyl, carbamoyl, alkylcarbamoyl, alkanoyl and alkoxycarbonyl groups.

Saturated hydrocarbon groups include C1-20 straight-chain, branched or cyclic alkyl groups, with C1-12 straight-chain, branched and cyclic alkyl groups being preferred. Examples of alkyl groups include methyl, ethyl, n-propyl, iso-propyl, n-butyl, iso-butyl, tert-butyl, 3-methylbutyl, pentyl, hexyl, 2-ethylhexyl, heptyl, octyl, nonyl, decyl, lauryl, cyclopropyl, cyclobutyl, cyclopentyl, cyclohexyl, cycloheptyl, cyclooctyl, cyclononyl and cyclododecyl. The same applies for groups comprising alkyl groups in their structures (for example, alkoxy, alkylamino and alkoxycarbonyl groups).

Unsaturated hydrocarbon groups include vinyl, 1-propenyl, allyl, propargyl, isopropenyl, 1-butenyl and 2-butenyl.

Alkanoyl groups include formyl, acetyl, propionyl, isobutyryl, valeryl and isovaleryl. The same applies for groups comprising alkanoyl groups in their structures (for example, alkanoyloxy and alkanoylamino groups). A "C1 alkanoyl group" is formyl, which also applies for groups containing alkanoyl groups in their structures.

In formula (I), Ar represents a trivalent aromatic hydrocarbon or trivalent heterocyclic group (which groups may be optionally substituted).

A trivalent aromatic hydrocarbon group is an atomic group remaining after removing 3 hydrogen atoms from a benzene ring or fused ring. The number of carbon atoms in the trivalent aromatic hydrocarbon group is preferably 6-60 and more preferably 6-20. Fused rings include naphthalene, anthracene, tetracene, pentacene, pyrene, perylene and fluorene. Of these, the trivalent aromatic hydrocarbon group is most preferably an atomic group remaining after removing 3 hydrogen atoms from a benzene ring. The aromatic hydrocarbon group may be optionally substituted. The numbers of carbon atoms of the substituents are not included in the number of carbon atoms in the trivalent aromatic hydrocarbon groups. Substituents include halogen atoms and saturated or unsaturated hydrocarbon, aryl, alkoxy, arylalkyl, aryloxy, monovalent heterocyclic, amino, nitro and cyano groups.

A trivalent heterocyclic group is an atomic group remaining after removing 3 hydrogens from a heterocyclic compound. The number of carbon atoms in the trivalent heterocyclic group is preferably 4-60 and more preferably 4-20. Examples of trivalent heterocyclic groups include atomic groups remaining after removing 3 hydrogens from a thiophene ring, thienothiophene ring, furan ring, pyrrole ring or pyridine ring. In particular, atomic groups remaining after removing 3 hydrogens from a thiophene ring or thienothiophene ring exhibit characteristic electrical properties and may be expected to also exhibit new electrical properties not found in the prior art. Trivalent aromatic heterocyclic groups are preferred as trivalent heterocyclic groups. The trivalent heterocyclic group may have substituents, and the numbers of carbons of the substituents are not included in the number of carbons in the trivalent heterocyclic group. Substituents include halogen atoms and saturated or unsaturated hydrocarbon, aryl, alkoxy, arylalkyl, aryloxy, monovalent heterocyclic, amino, nitro and cyano groups.

A heterocyclic compound referred to here is an organic compound with a ring structure wherein the elements composing the ring include not only carbon but also heteroatoms such as oxygen, sulfur, nitrogen, phosphorus, boron and silicon.

In formula (II), A' represents hydrogen, a halogen atom or a monovalent group, and when multiple A' groups are present, they may be the same or different, and at least one A' is an electron-withdrawing group. Preferably, two A' groups are both electron-withdrawing groups, since this will allow the LUMO to be further lowered. Examples of electron-withdrawing groups include the same groups mentioned above as electron-withdrawing groups for A, with cyano, nitro and halogen atoms being preferred, and cyano being more preferred. Most preferably, the group represented by formula (II) is one in which both of the A' groups are cyano groups.

In formula (II), Ar' represents an optionally substituted C6 or greater divalent aromatic hydrocarbon or an optionally substituted C4 or greater divalent heterocyclic group.

A divalent aromatic hydrocarbon group is an atomic group remaining after removing two hydrogen atoms from a benzene ring or fused ring. The number of carbon atoms in the divalent aromatic hydrocarbon group is preferably 6-60 and more preferably 6-20. Fused rings include naphthalene, anthracene, tetracene, pentacene, pyrene, perylene and fluorene. Particularly preferred among these are atomic groups remaining after removing 2 hydrogen atoms from a benzene ring. The aromatic hydrocarbon groups may be optionally substituted. The numbers of carbon atoms of the substituents are not included in the number of carbon atoms in the divalent aromatic hydrocarbon groups. Substituents include halogen atoms and saturated or unsaturated hydrocarbon, aryl, alkoxy, arylalkyl, aryloxy, monovalent heterocyclic, amino, nitro and cyano groups.

A divalent heterocyclic group is an atomic group remaining after removing two hydrogens from a heterocyclic compound. The number of carbon atoms in the divalent heterocyclic group is preferably 4-60 and more preferably 4-20. Examples of divalent heterocyclic groups include atomic groups remaining after removing 2 hydrogens from a thiophene ring, thienothiophene ring, furan ring, pyrrole ring or pyridine ring. In particular, atomic groups remaining after removing 2 hydrogens from a thiophene ring or thienothiophene ring exhibit characteristic electrical properties and may be expected to also exhibit new electrical properties not found in the prior art. Divalent aromatic heterocyclic groups are preferred as divalent heterocyclic groups. The divalent heterocyclic group may have substituents, and the numbers of carbons of the substituents are not included in the number of carbons in the divalent heterocyclic group. Substituents include halogen atoms and saturated or unsaturated hydrocarbon, aryl, alkoxy, arylalkyl, aryloxy, monovalent heterocyclic, amino, nitro and cyano groups.

In formula (II), A' represents hydrogen, a halogen atom or a monovalent group, and R¹ and R² are the same or different and each represents hydrogen, a halogen atom or a monovalent group. Examples of monovalent groups in such groups include the same monovalent groups mentioned for A in formula (I).

In particular, R¹ and R² are preferably fluorine, C1-20 alkyl, C1-20 fluoroalkyl, C1-20 alkoxy or C1-20 fluoroalkoxy groups, and more preferably fluorine, C1-20 alkyl or C1-20 fluoroalkyl groups, since these will allow the LUMO to be lowered even further. Either or both R¹ and R² are preferably fluorine.

The group represented by formula (I) is preferably a group represented by formula (III), since this will allow the LUMO of the conjugated compound to be lowered still further.

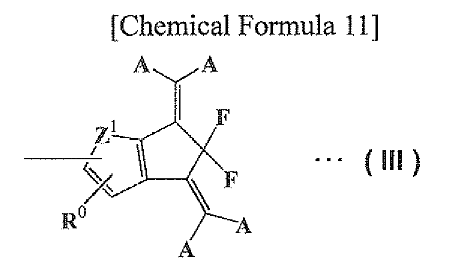

In formula (III), A has the same definition as A in formula (I), and multiple A groups may be the same or different, with at least one A being an electron-withdrawing group. R⁰ represents hydrogen, C1-20 alkyl, C1-20 fluoroalkyl, C1-20 alkoxy or C1-20 fluoroalkoxy, Z¹ represents any group represented by formulas (i)-(ix), R³, R⁴, R⁵ and R⁶ are the same or different and each represents hydrogen or a monovalent group, and R³ and R⁴ may be bonded together to form a ring. Z¹ is preferably a group represented by formula (ii).

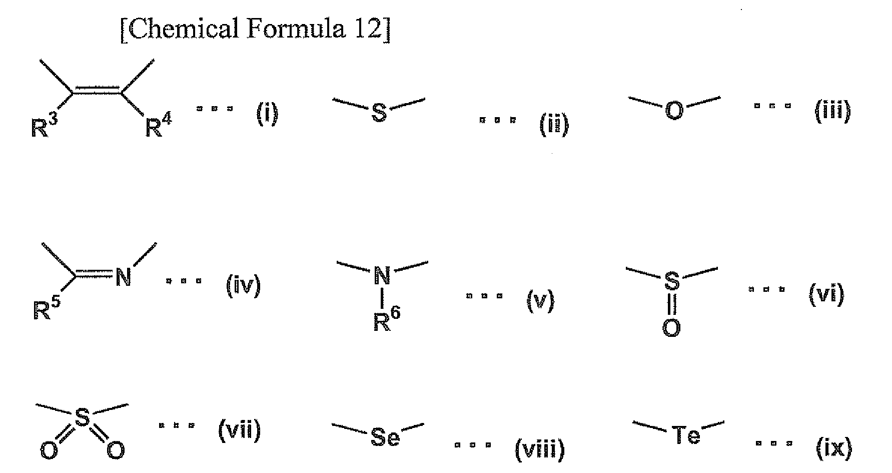

On the other hand, the group represented by formula (II) is preferably a group represented by formula (V), since this will allow the LUMO of the conjugated compound to be lowered still further.

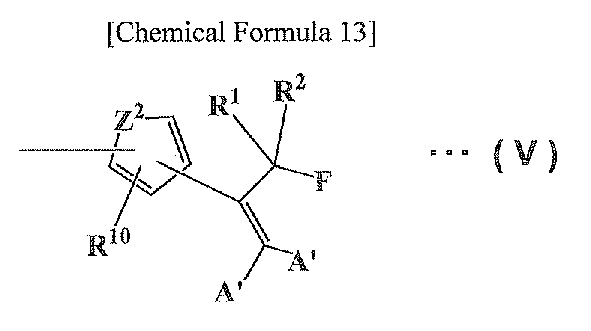

In formula (V), A', R¹ and R² have the same definitions as A', R¹ and R² in formula (II), and multiple A' groups may be the same or different, with at least one A' being an electron-withdrawing group. R¹⁰ represents hydrogen, C1-20 alkyl, C1-20 fluoroalkyl, C1-20 alkoxy or C1-20 fluoroalkoxy, Z² represents any group represented by formulas (xi)-(xix), R¹³, R¹⁴, R¹⁵ and R¹⁶ are the same or different and each represents hydrogen or a monovalent group, and R¹³ and R¹⁴ may be bonded together to form a ring. Z² is preferably a group represented by formula (xi) or (xii), and more preferably a group represented by formula (xii).

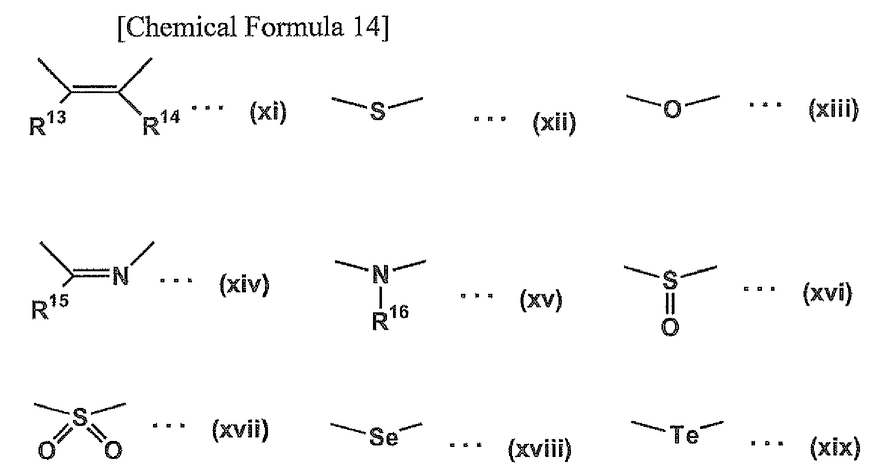

Alkyl groups for R⁰ and R¹⁰ include C1-20 straight-chain, branched or cyclic alkyl groups, with C1-12 straight-chain, branched and cyclic alkyl groups being preferred. Examples of alkyl groups include methyl, ethyl, n-propyl, iso-propyl, n-butyl, iso-butyl, tert-butyl, 3-methylbutyl, pentyl, hexyl, 2-ethylhexyl, heptyl, octyl, nonyl, decyl, lauryl, cyclopropyl, cyclobutyl, cyclopentyl, cyclohexyl, cycloheptyl, cyclooctyl, cyclononyl and cyclododecyl. Alkoxy groups include C1-20 alkoxy groups comprising these alkyl groups in their structures. Alkoxy groups include C1-20 straight-chain, branched or cyclic alkoxy groups comprising these alkyl groups in their structures, with those comprising C1-12 straight-chain, branched and cyclic alkyl groups being preferred. Fluoroalkyl groups for R¹⁰ include the aforementioned alkyl groups having some or all of their hydrogens replaced with fluorine atoms, with C1-12 straight-chain, branched and cyclic fluoroalkyl groups being preferred. Fluoroalkoxy groups include C1-20 fluoroalkoxy groups comprising these fluoroalkyl groups in their structures, with those comprising C1-12 straight-chain, branched and cyclic fluoroalkyl groups being preferred.

Monovalent groups for R³, R⁴, R⁵, R⁶, R¹³, R¹⁴, R¹⁵ and R¹⁶ include straight-chain or branched low molecular chains, C3-60 monovalent cyclic groups (monocyclic or fused rings, carbon rings or heterocyclic rings, saturated or unsaturated, and optionally substituted), saturated or unsaturated hydrocarbon groups, alkyl groups substituted with hydroxyl, alkoxy, alkanoyloxy, amino, oxyamino, alkylamino, dialkylamino, alkanoylamino, cyano, nitro, sulfo and halogen atoms, alkoxysulfonyl groups (wherein the hydrogens of the alkoxy groups may be substituted with halogen atoms), alkylsulfonyl groups (wherein the hydrogens of the alkyl groups may be substituted with halogen atoms), sulfamoyl, alkylsulfamoyl, carboxyl, carbamoyl, alkylcarbamoyl, alkanoyl and alkoxycarbonyl groups.

As alkyl groups there may be mentioned C1-20 straight-chain, branched or cyclic alkyl groups, with C1-12 straight-chain, branched and cyclic alkyl groups being preferred. Examples of alkyl groups include methyl, ethyl, n-propyl, iso-propyl, n-butyl, iso-butyl, tert-butyl, 3-methylbutyl, pentyl, hexyl, 2-ethylhexyl, heptyl, octyl, nonyl, decyl, lauryl, cyclopropyl, cyclobutyl, cyclopentyl, cyclohexyl, cycloheptyl, cyclooctyl, cyclononyl and cyclododecyl. The same applies for groups comprising alkyl groups in their structures (for example, alkoxy, alkylamino and alkoxycarbonyl groups).

Examples of unsaturated hydrocarbon groups include vinyl, 1-propenyl, allyl, propargyl, isopropenyl, 1-butenyl and 2-butenyl.

Alkanoyl groups include formyl, acetyl, propionyl, isobutyryl, valeryl and isovaleryl. The same applies for groups comprising alkanoyl groups in their structures (for example, alkanoyloxy and alkanoylamino groups). A "C1 alkanoyl group" is formyl, which also applies for groups containing alkanoyl groups in their structures.

The conjugated compound has a group represented by formula (I) and/or formula (II), and from the viewpoint of allowing an even lower LUMO to be obtained for an increased electron transport property, it is preferably one having 2 or more groups represented by formula (I), one having 2 or more groups represented by formula (II), or one having a group represented by formula (I) and a group represented by formula (II).

From the viewpoint of allowing the LUMO to be lowered even further, the conjugated compound is preferably one having a group represented by formula (III) as the group represented by formula (1), and most preferably one having 2 or more groups represented by formula (III). There are preferred compounds having groups represented by formula (V) as the groups represented by formula (II), and more preferred are those having 2 or more groups represented by formula (V).

As conjugated compounds having 2 or more groups represented by formula (III) there are preferred conjugated compounds represented by formula (IV), since they have even more excellent electron transport properties as organic n-type semiconductors.

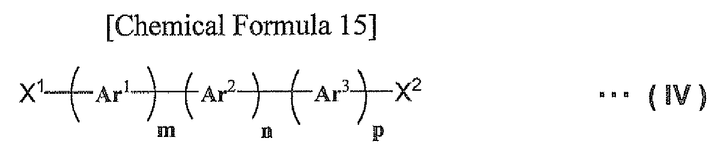

In formula (IV), X¹ and X² are the same or different and each is a group represented by formula (III). Ar¹, Ar² and Ar³ are the same or different and each represents an optionally substituted C6 or greater divalent aromatic hydrocarbon or optionally substituted C4 or greater divalent heterocyclic group, and m, n and p are the same or different and each represents an integer of 0-6. This is with the proviso that m+n+p is an integer of 1 or greater.

As conjugated compounds having 2 or more groups represented by formula (V) there are preferred conjugated compounds represented by formula (VI), since they have even more excellent electron transport properties as organic n-type semiconductors.

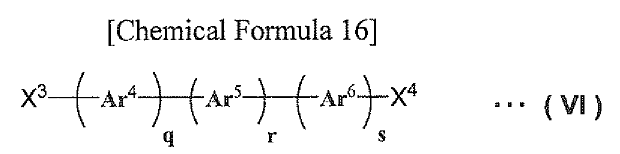

In formula (VI), X³ and X⁴ are the same or different and each is a group represented by formula (V). Ar⁴, Ar⁵ and Ar⁶ are the same or different and each represents an optionally substituted C6 or greater divalent aromatic hydrocarbon or optionally substituted C4 or greater divalent heterocyclic group, and q, r and s are the same or different and each represents an integer of 0-6. This is with the proviso that q+r+s is an integer of 1 or greater.

C6 or greater divalent aromatic hydrocarbon and C4 or greater divalent heterocyclic groups for Ar¹, Ar², Ar³, Ar⁴, Ar⁵ and Ar⁶ include the same groups as those mentioned above for Ar', with thienylene being preferred.

These conjugated compounds are expected to have high electron transport properties as organic n-type semiconductors. For an increased effect, they are preferably compounds that readily adopt a π-π stack structure, which increases the planarity of the π-conjugated structure other than the groups represented by formula (I) or (II). From this viewpoint, Ar¹, Ar² and Ar³ in formula (IV) and Ar⁴, Ar⁵ and Ar⁶ in formula (VI) preferably have structures comprising fused rings or thiophene rings. A structure comprising a thiophene backbone is especially preferred since the plane spacing in the π-π stack structure can be reduced. From the viewpoint of improving the solubility in organic solvents and maintaining π-conjugated planarity, at least one of Ar¹, Ar² and Ar³ preferably has a substituent, and preferably at least one of Ar⁴, Ar⁵ and Ar⁶ has a substituent.

Compounds represented by formula (IV) or (VI) include compounds represented by the following formulas (1) to (21).

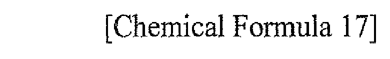

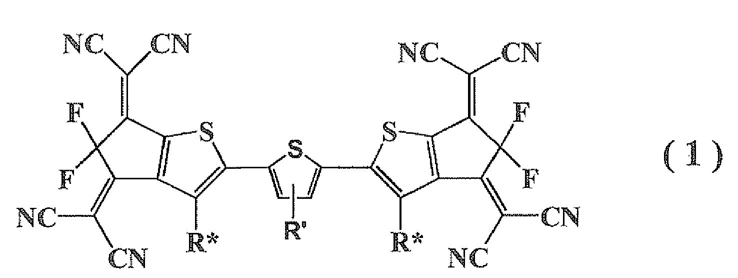

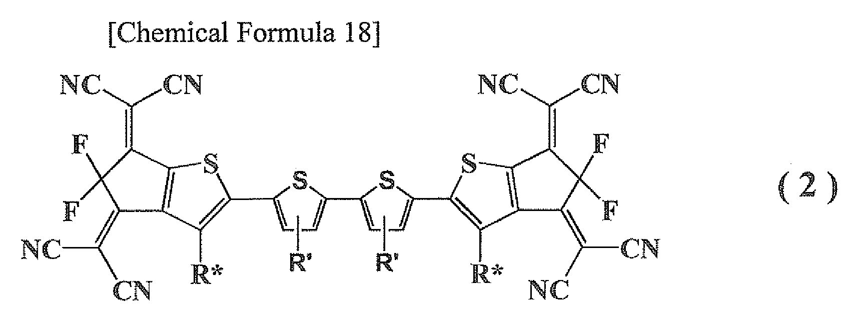

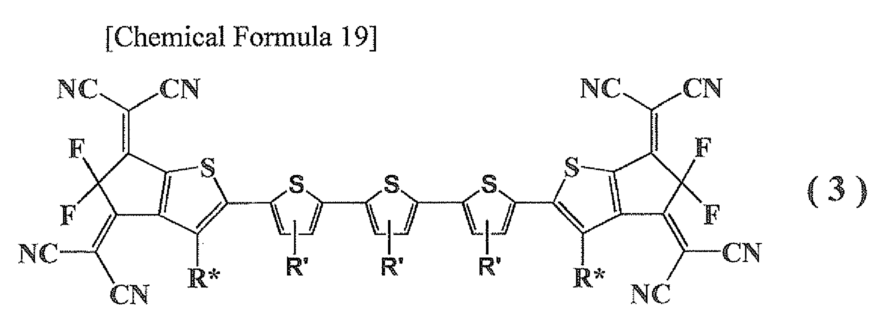

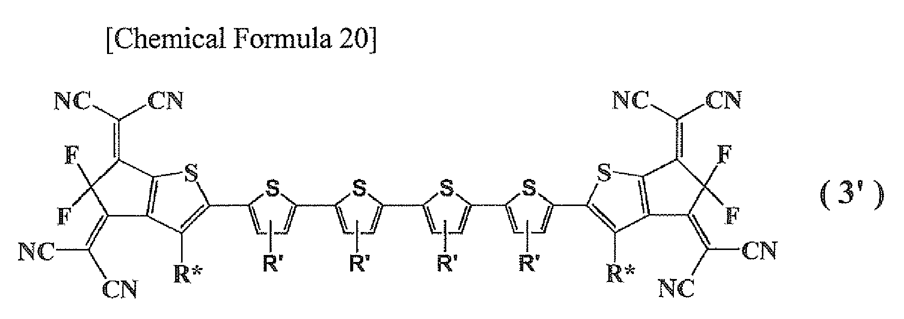

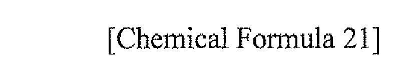

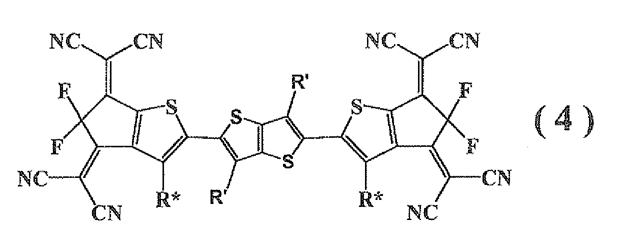

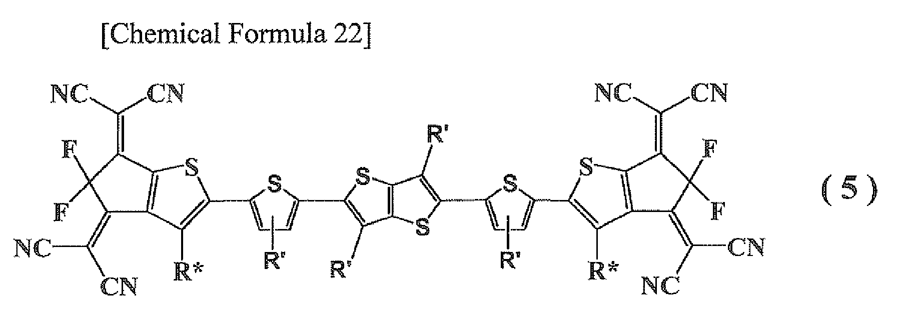

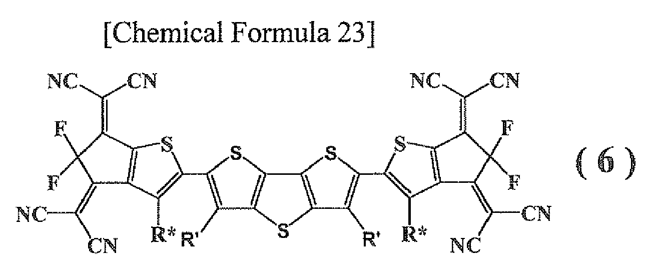

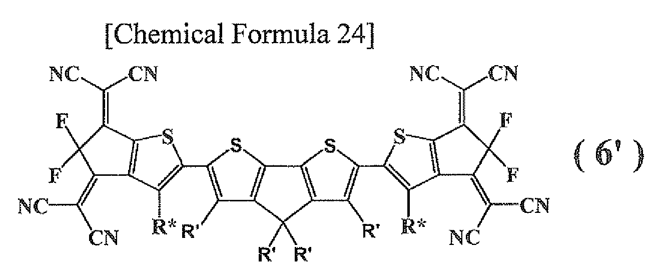

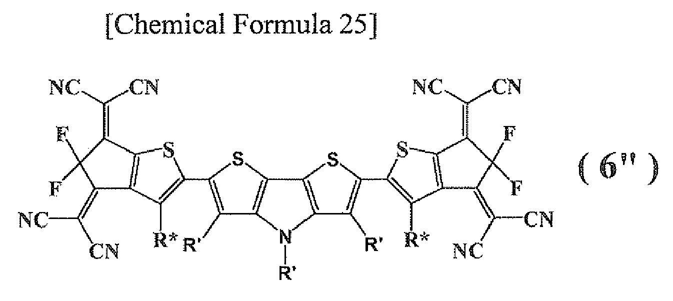

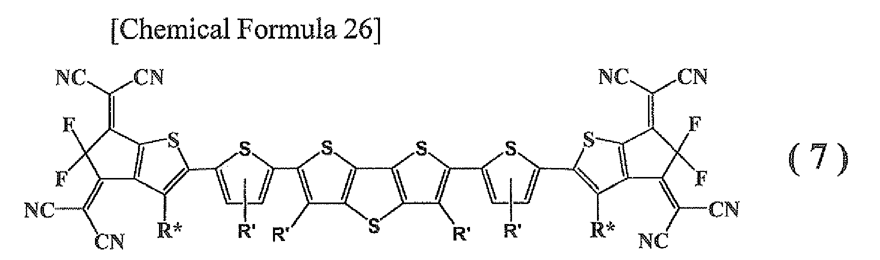

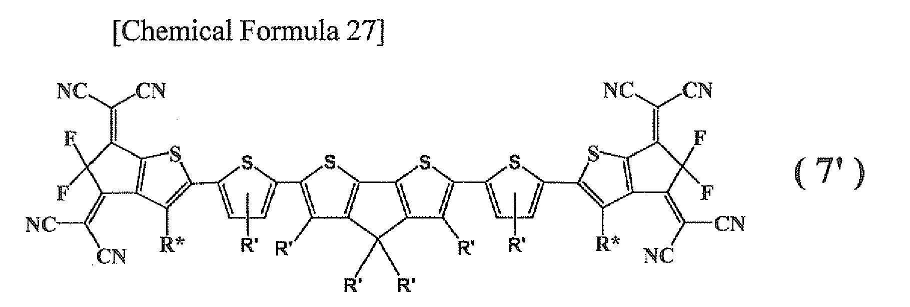

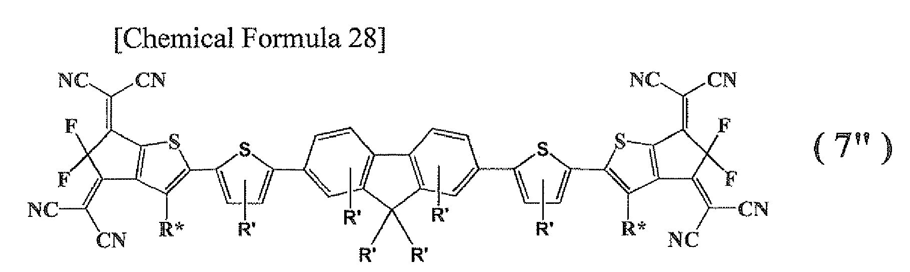

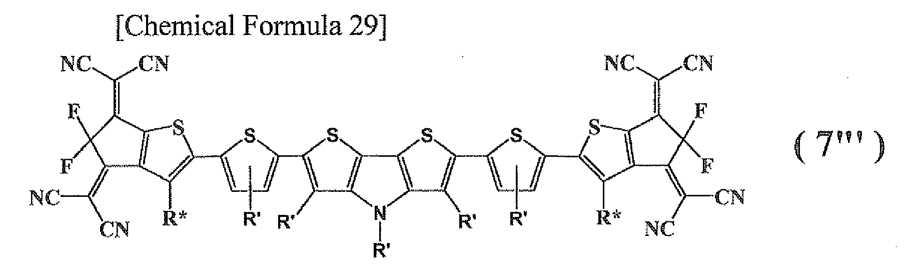

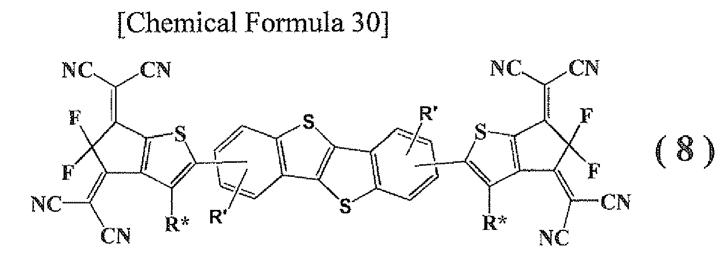

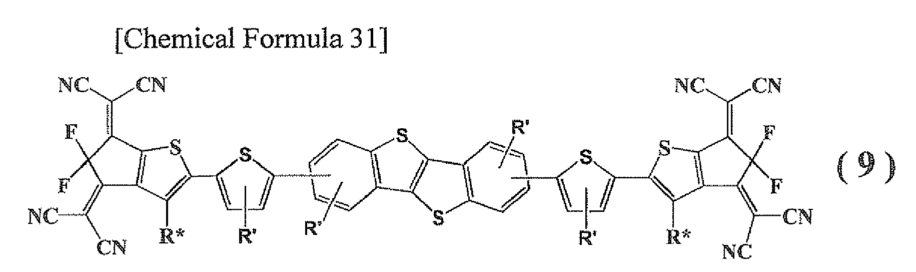

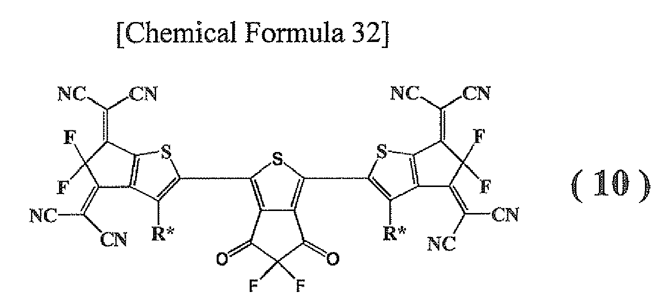

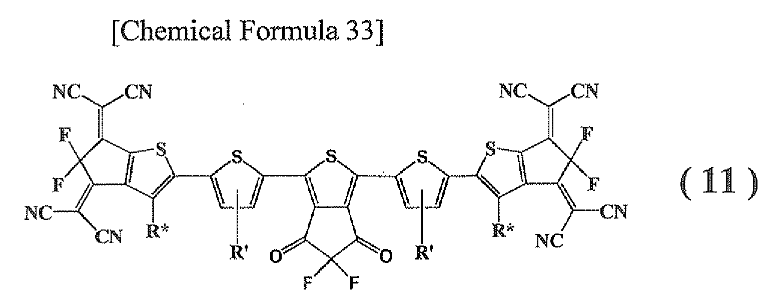

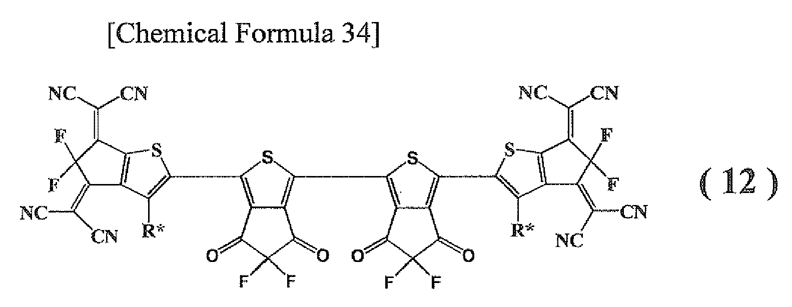

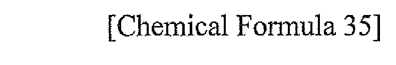

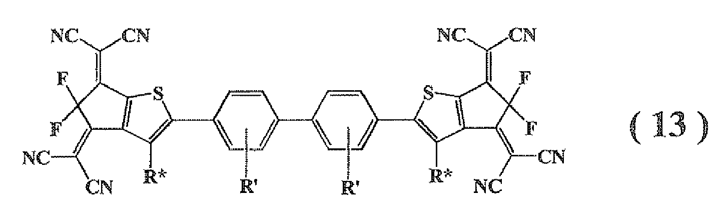

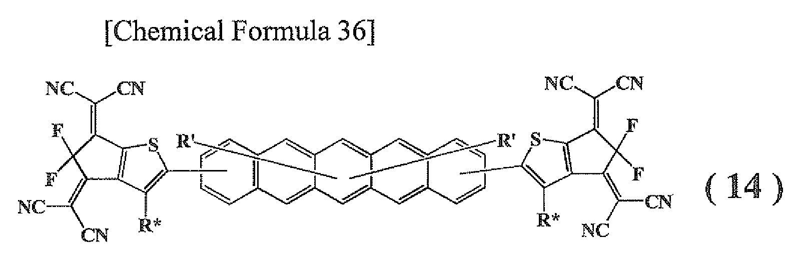

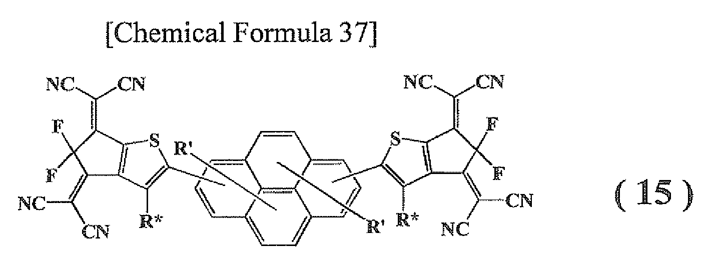

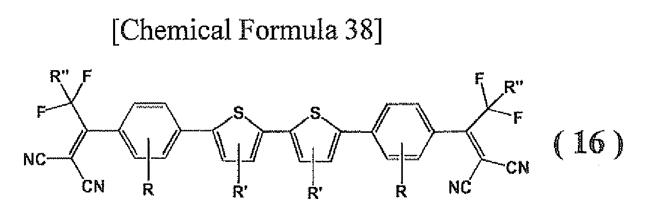

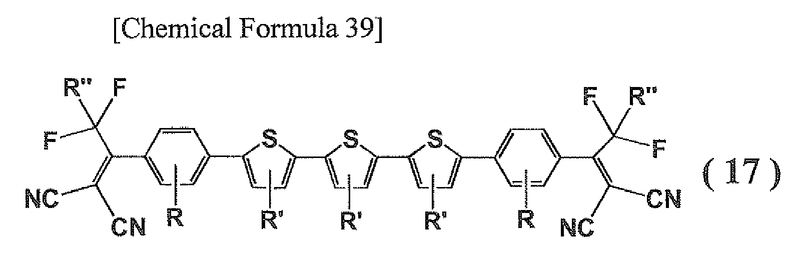

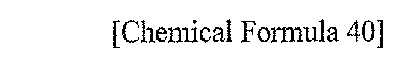

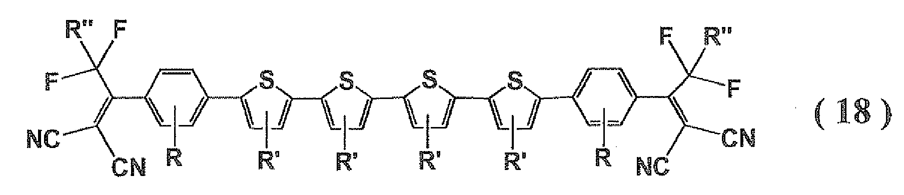

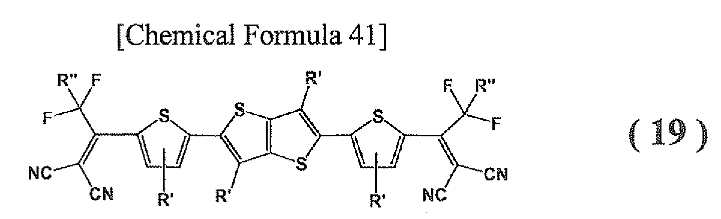

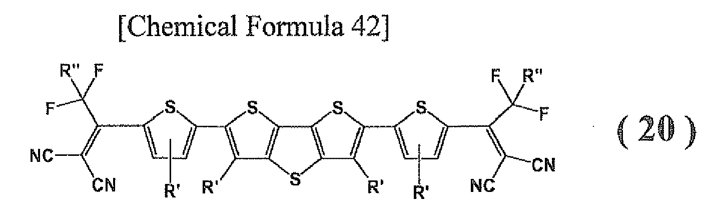

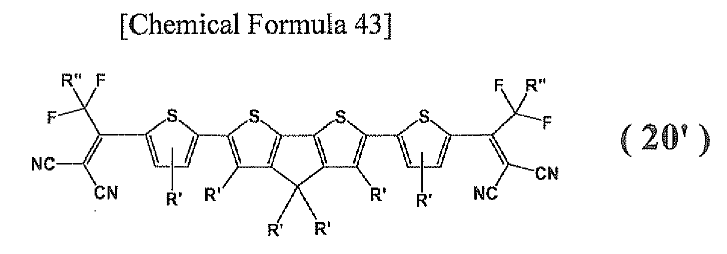

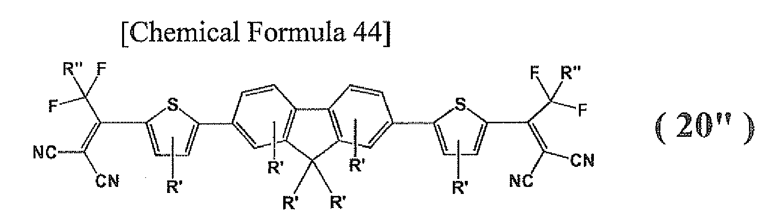

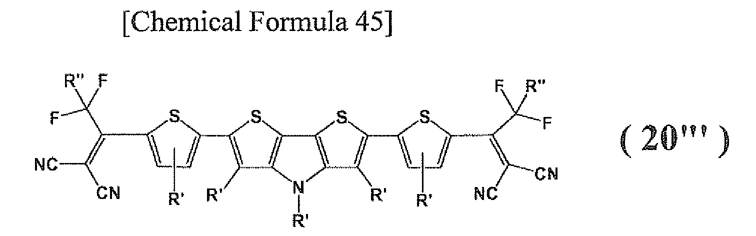

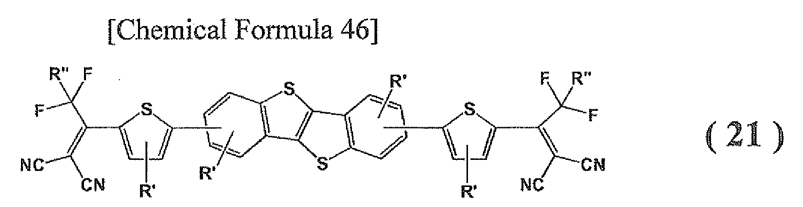

In formulas (1), (2), (3), (3'), (4), (5), (6), (6'), (6"), (7), (7'), (7"), (7"'), (8), (9), (10), (11), (12), (13), (14), (15), (16), (17), (18), (19), (20), (20'), (20"), (20"') and (21), the groups represent hydrogen, C1-20 alkyl or C1-20 alkoxy groups. Also, R^{*}, R' and R" are the same or different and each represents hydrogen, fluorine, C1-20 alkyl, C1-20 fluoroalkyl, C1-20 alkoxy or C1-20 fluoroalkoxy. Multiple R, R^{*}, R' and R" groups in the molecule may be the same or different. Of these, R, R^{*} and R' are preferably hydrogen or C1-20 alkyl groups, and R" is preferably a fluorine atom or C1-20 fluoroalkyl group.

The conjugated compounds have a reduction potential based on ferrocene, as determined by electrochemical measurement (cyclic voltammetry), of preferably -2.0 V to +0.5 V and more preferably -1.8 V to +0.2 V. If the reduction potential is within this numerical range, the conjugated compound will be sufficiently suitable as an n-type semiconductor with excellent electron injection and excellent electron transport properties. The reduction potential can be measured by the following method, for example.

For measurement of the reduction potential, an organic solvent is prepared containing about 0.1 mol/L tetrabutylammonium perchlorate and tetrabutylammonium hexafluorophosphate, as supporting electrolytes, and the material to be measured is dissolved therein to about 0.1-2 mM. The oxygen is removed from the obtained solution by a method such as dry nitrogen bubbling, vacuum deaeration or ultrasonic irradiation. Next, a platinum electrode or glassy carbon electrode is used as the work electrode with a platinum electrode as the counter electrode, for electrolytic reduction from an electrically neutral state at a sweep rate of 100 mV/sec. The potential of the first peak value detected during electrolytic reduction is compared with the oxidation-reduction potential of a reference material such as ferrocene, to obtain the oxidation (or reduction) potential for the material being measured. The value of the oxidation (or reduction) potential obtained in this manner, converted based on ferrocene, is used as the reduction potential.

A preferred method for producing the conjugated compounds described above will now be explained. The conjugated compounds can be produced by reacting compounds represented by the following formulas (VII), (VIII), (IX), (X), (XI), (XII) and (XIII) (hereunder also abbreviated as "(VII)-(XIII)") as starting materials. More specifically, compounds represented by formulas (VII)-(X) are preferably produced by reacting compounds represented by formulas (XI)-(XIII). In this case, W¹ in a compound represented by one of formulas (VII)-(X) reacts with W¹ and W² in a compound represented by one of formulas (XI)-(XIII), forming a bond and thus producing a conjugated compound.

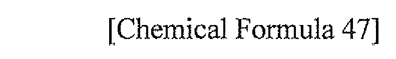

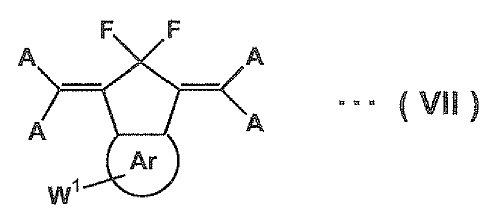

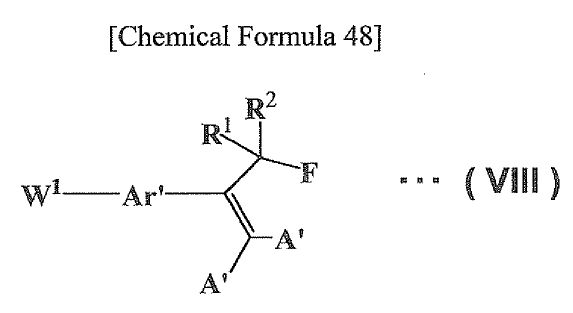

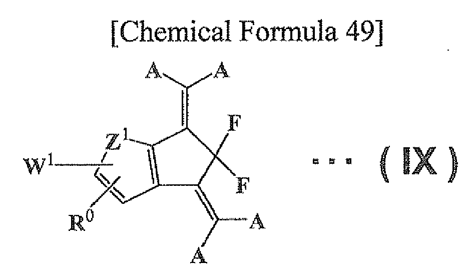

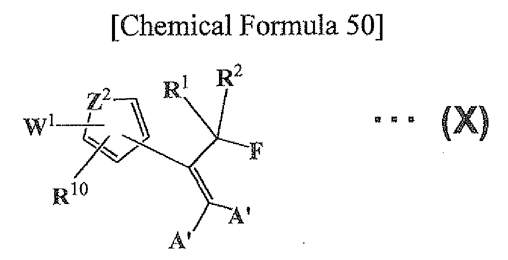

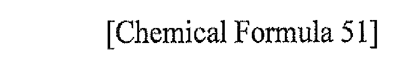

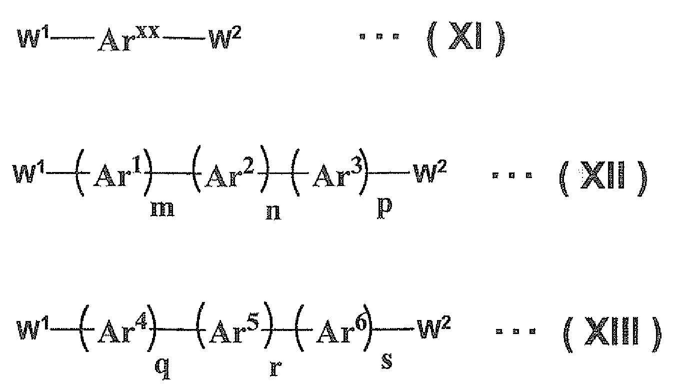

In formulas (VII)-(XIII), A, A', Ar, Ar', Ar¹, Ar², Ar³, Ar⁴, Ar⁵, Ar⁶, Z¹, Z², R⁰,R¹⁰, R¹, R², m, n, p, q, r and s have the same definitions as above. Ar^{XX} represents any of Ar¹-Ar⁶. W¹ and W² are the same or different, and each represents hydrogen, a halogen atom, or an alkyl sulfonate, aryl sulfonate, arylalkyl sulfonate, boric acid ester residue, sulfoniummethyl, phosphoniummethyl, phosphonatemethyl, monohalogenated methyl group, boric acid residue (-B(OH)₂), formyl, trialkylstannyl or vinyl group.

From the viewpoint of facilitating synthesis and reaction of the compounds represented by formulas (VII)-(XIII), W¹ and W² are preferably the same or different groups from among halogen atoms and alkyl sulfonate, aryl sulfonate, arylalkyl sulfonate, boric acid ester residue, boric acid residue and trialkylstannyl groups.

Examples of boric acid ester residues include groups represented by the following formulas.

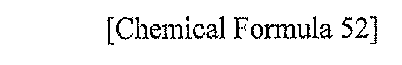

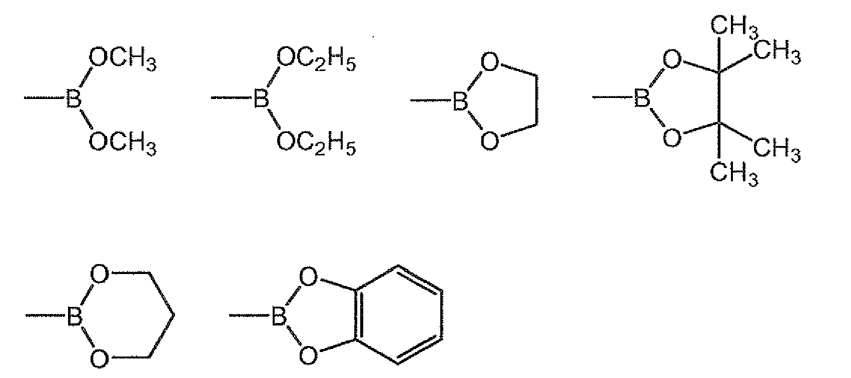

Processes for using the aforementioned starting materials to produce conjugated compounds include a process employing Suzuki coupling reaction, a process employing Grignard reaction, a process employing Stille reaction, a process employing a Ni(O) catalyst, a process employing an oxidizing agent such as FeCl₃, a process employing anionic oxidation reaction, a process employing palladium acetate and an organic base, a process involving preparation of a lithiated compound from an α-unsubstituted or halogenated derivative of the starting compound, and oxidative coupling, a process employing electrochemical oxidation reaction, and a process involving decomposition of an intermediate compound with an appropriate leaving group. From the viewpoint of improving the reaction yield, microwave irradiation may be employed when such methods are used.

Of these, processes employing Suzuki coupling reaction, processes employing Grignard reaction, processes employing Stille reaction, processes employing Ni(O) catalysts, processes employing anionic oxidation reaction and processes employing palladium acetate and organic bases are preferred for easier structural control, ready availability and simplification of the reaction procedure.

The catalyst used for Suzuki coupling reaction may be tetrakis(triphenylphosphine)palladium or palladium acetate, with addition of at least one equivalent and preferably 1-10 equivalents of an inorganic base such as potassium carbonate, sodium carbonate or barium hydroxide, an organic base such as triethylamine or an inorganic salt such as cesium fluoride, with respect to the monomer. The reaction may be carried out in a two-phase system, with the inorganic salt in aqueous solution. The solvent used for the reaction may be N,N-dimethylformamide, toluene, dimethoxyethane, tetrahydrofuran or the like. The reaction temperature will depend on the solvent used but is preferably 50-160°C. The temperature may be increased to near the boiling point of the solvent for reflux. The reaction time will be between 1 and 200 hours. Suzuki coupling reaction may be carried out by the method described in Chem. Rev. Vol. 95, p.2457(1995).

For reaction using a Ni(O) catalyst, the process may employ a zerovalent nickel complex as the Ni(O) catalyst, or it may include reacting a nickel salt in the presence of a reducing agent to produce zerovalent nickel in the system. Examples of zerovalent nickel complexes include bis(1,5-cyclooctadiene)nickel(0), (ethylene)bis(triphenylphosphine)nickel(0) and tetrakis(triphenylphosphine)nickel. Among these, bis(1,5-cyclooctadiene)nickel(0) is preferred from the viewpoint of excellent general utility and economy.

Addition of a neutral ligand during the reaction is also preferred from the viewpoint of increasing the yield. A "neutral ligand" is a ligand containing no anions or cations, and examples thereof include nitrogen-containing ligands such as 2,2'-bipyridyl, 1,10-phenanthroline, methylenebisoxazoline and N,N'-tetramethylethylenediamine; and tertiary phosphine ligands such as triphenylphosphine, tritolylphosphine, tributylphosphine and triphenoxyphosphine. Nitrogen-containing ligands are preferred from the viewpoint of greater flexibility and lower cost, while 2,2'-bipyridyl is more preferred from the viewpoint of higher reactivity and yield. From the viewpoint of improving the conjugated compound yield, it is preferred to add 2,2'-bipyridyl as a neutral ligand to a system containing bis(1,5-cyclooctadiene)nickel(0) for reaction employing a Ni(0) catalyst. As nickel salts in the process for producing zerovalent nickel in the system, there may be used nickel chloride and nickel acetate. Reducing agents include zinc, sodium hydride, hydrazine and their derivatives, and also lithium aluminum hydride. Ammonium iodide, lithium iodide, potassium iodide and the like may also be used as additives.

For Stille reaction, the catalyst used may be tetrakis(triphenylphosphine)palladium or palladium acetate, and reaction may be conducted using an organic tin compound as monomer. The solvent used for the reaction may be N,N-dimethylformamide, toluene, dimethoxyethane, tetrahydrofuran or the like. The reaction temperature will depend on the solvent used but is preferably 50-160°C. The temperature may be increased to near the boiling point of the solvent for reflux. The reaction time is preferably between 1 and 200 hours.

For a process employing anionic oxidation reaction, a halogen-substituted starting compound, or the starting compound itself, may be used as the monomer for reaction with n-butyllithium to prepare a lithiated derivative, which is then treated with an oxidizing agent such as copper(II) bromide, copper(II) chloride, iron(III) acetylacetonate or the like. The solvent used for the reaction may be toluene, dimethoxyethane, tetrahydrofuran, hexane, heptane, octane or the like. The reaction temperature will also depend on the solvent used but is preferably 50-160°C. The temperature may be increased to near the boiling point of the solvent for reflux. The reaction time will be between about 5 minutes and 200 hours.

For a process employing palladium acetate and an organic base, a halogen-substituted compound may be used as the monomer and palladium(II) acetate and an organic base such as diisopropylamine or triethylamine added for reaction. The solvent used for the reaction may be N,N-dimethylforinamide, toluene, dimethoxyethane, tetrahydrofuran or the like. The reaction temperature will also depend on the solvent used but is preferably 50-160°C. The temperature may be increased to near the boiling point of the solvent for reflux. The reaction time will be between about 5 minutes and 200 hours.

A conjugated compound as mentioned above may be produced, for example, by reacting a compound represented by any of formulas (XIV), (XV), (XVI), (XVII), (XVIII), (XIX), (XX) and (XXI) (hereunder also abbreviated as "(XIV)-(XXI)") with a compound represented by any of formulas (XI)-(XIII), as starting materials, to obtain a synthetic intermediate, and then further reacting the synthetic intermediate.

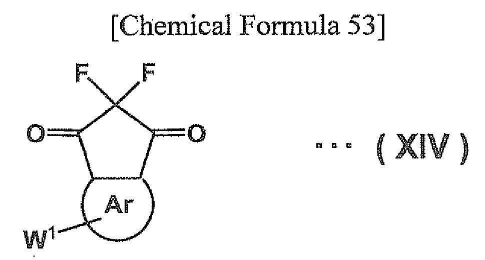

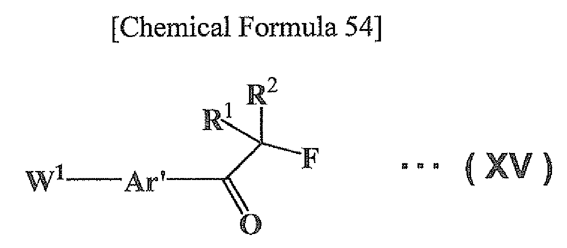

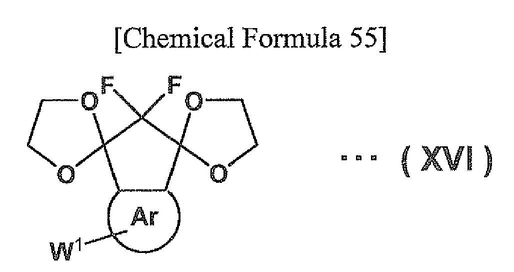

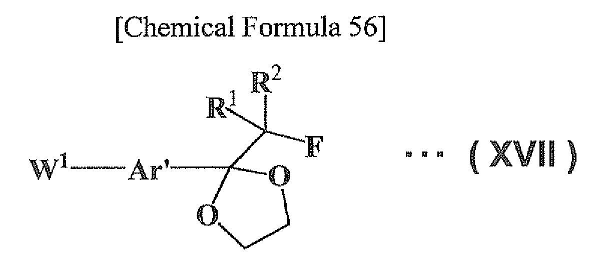

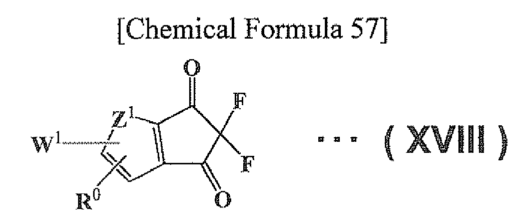

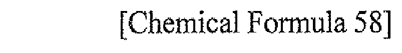

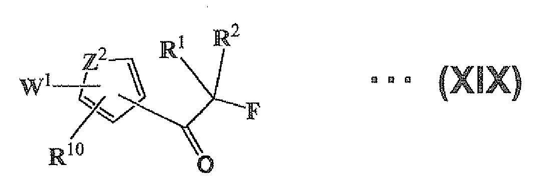

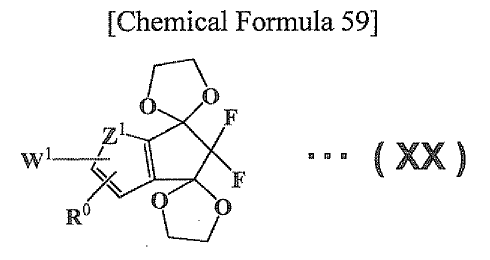

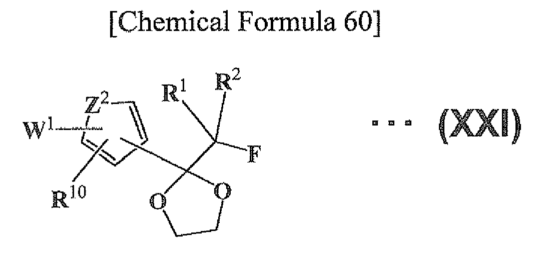

In formulas (XIV)-(XXI), W¹, Ar, Ar', Ar¹, Ar², Ar³, Z¹, Z², R⁰, R¹⁰, R¹, R², m, n and p have the same definitions as above. The reaction between the compound represented by formulas (XIV)-(XXI) and the compound represented by formulas (XI)-(XIII) may be conducted in the same manner as the reaction between the compound represented by formulas (VII)-(X) and the compound represented by formulas (XI)-(XIII).

In a method for obtaining such a synthetic intermediate, a compound represented by formula (XX) may be reacted with a compound represented by formula (XII), for example, to produce a compound represented by formula (XXII) as an intermediate. After the reaction, the alkylenedioxy group in the intermediate may be converted to a carboxyl group, and the carboxyl group further converted to a group represented by "=CA₂" (where A has the same definition as above) by a known method, to produce a conjugated compound represented by formula (IV).

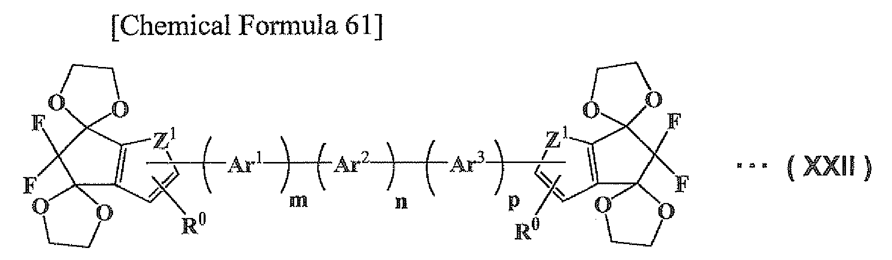

In formula (XXII), Z¹, R⁰, Ar¹, Ar², Ar³, m, n and p have the same definitions as above.

When a conjugated compound is to be used as a material for an organic thin-film element, it is preferably subjected to purification treatment by a method such as sublimation purification or recrystallization, since the purity will affect the element characteristics.

An organic thin-film according to the invention will now be explained. The organic thin-film of this embodiment is one comprising a conjugated compound as described above.

The organic thin-film may be one comprising only one of the aforementioned conjugated compounds, or it may include two or more of such conjugated compounds. In order to enhance the electron transport and hole transport properties of the organic thin-film, a low molecular compound or high molecular compound having an electron transport or hole transport property (an electron transport material or hole transport material) may also be combined in addition to the conjugated compound.

Any known hole transport material may be used, examples of which include pyrazolines, arylamines, stilbenes, triaryldiamines, oligothiophenes, polyvinylcarbazoles, polysilanes, polysiloxanes with aromatic amines on the side chains or main chain, polyanilines, polythiophenes, polypyrroles, polyarylenevinylenes and polythienylenevinylenes, as well as derivatives of the foregoing.

Any known electron transport materials may also be used, examples of which include metal complexes of oxadiazoles, quinodimethanes, benzoquinones, naphthoquinones, anthraquinones, tetracyanoanthraquinodimethanes, fluorenones, diphenyldicyanoethylenes, diphenoquinones and 8-hydroxyquinolines, polyquinolines, polyquinoxalines, polyfluorenes, C₆₀ and other fullerenes, and derivatives of the foregoing.

The organic thin-film may also contain a charge generating material for generation of an electrical charge upon absorption of light in the organic thin-film. Any known charge generating materials may be used, examples of which include azo compounds, diazo compounds, ametallic phthalocyanine compounds, metal phthalocyanine compounds, perylene compounds, polycyclic quinone-based compounds, squarylium compounds, azulenium compounds, thiapyrylium compounds or C₆₀ and other fullerenes.

The organic thin-film may also contain materials necessary for exhibiting various functions. Examples of such materials include sensitizing agents to enhance the function of generating charge by light absorption, stabilizers to increase stability, and UV absorbers for absorption of UV light.

The organic thin-film may also contain high molecular materials as macromolecular binders in addition to the compounds mentioned above, in order to improve the mechanical properties. As macromolecular binders there are preferably used ones that do not interfere with the electron transport or hole transport property, and ones with weak absorption for visible light.

Examples of such macromolecular binders include poly(N-vinylcarbazole), polyaniline, polythiophene, poly(p-phenylenevinylene), poly(2,5-thienylenevinylene), polycarbonate, polyacrylate, polymethyl acrylate, polymethyl methacrylate, polystyrene, polyvinyl chloride, polysiloxane, and derivatives of the foregoing.

For production of the organic thin-film there may be employed a method of film formation from a solution comprising the conjugated compound and, as necessary, an electron transport material or hole transport material and a macromolecular binder and solvent in admixture therewith. When the conjugated compound is sublimating, it can be formed into a thin-film by a vacuum vapor deposition method.

The solvent may be any one that dissolves the conjugated compound and the electron transport material or hole transport material and macromolecular binders combined therewith.

Such solvents include unsaturated hydrocarbon-based solvents such as toluene, xylene, mesitylene, tetralin, decalin, bicyclohexyl, n-butylbenzene, sec-butylbenzene and tert-butylbenzene, halogenated saturated hydrocarbon-based solvents such as carbon tetrachloride, chloroform, dichloromethane, dichloroethane, chlorobutane, bromobutane, chloropentane, bromopentane, chlorohexane, bromohexane, chlorocyclohexane and bromocyclohexane, halogenated unsaturated hydrocarbon-based solvents such as chlorobenzene, dichlorobenzene and trichlorobenzene, and ether-based solvents such as tetrahydrofuran and tetrahydrropyran. The conjugated compound may be dissolved in such solvents to at least 0.1 wt% for most purposes, although this will differ depending on the structure and molecular weight of the compound.

The method for forming the film may be a coating method such as spin coating, casting, microgravure coating, gravure coating, bar coating, roll coating, wire bar coating, dip coating, spray coating, screen printing, flexographic printing, offset printing, ink jet printing, dispenser printing, nozzle coating or capillary coating. Particularly preferred are spin coating, flexographic printing, ink jet printing, dispenser printing, nozzle coating and capillary coating.

The film thickness of the organic thin-film is preferably about 1 nm-100 µm, more preferably 2 nm-1000 nm, even more preferably 5 nm-500 nm and most preferably 20 nm-200 nm.

The step of producing the organic thin-film may also include a step of orienting the conjugated compound. An organic thin-film having the conjugated compound oriented by such a step will have the main chain molecules or side chain molecules aligned in a single direction, thus improving the electron mobility or hole mobility.

The method of orienting the conjugated compound may be a known method for orienting liquid crystals. Rubbing, photoorientation, shearing (shear stress application) and pull-up coating methods are convenient, useful and easy orienting methods, and rubbing and shearing are preferred.

Since the organic thin-film has an electron transport property or hole transport property, the transport of electrons or holes introduced from the electrode or charge generated by photoabsorption can be controlled for use in various organic thin-film elements such as organic thin-film transistors or organic photoelectric conversion elements (organic solar cells, optical sensors and the like). When an organic thin-film of the invention is used in such organic thin-film elements, it is preferably used after orientation by orienting treatment in order to further enhance the electron transport property or hole transport property.

Application of an organic thin-film as described above to an organic thin-film transistor will now be explained. The organic thin-film transistor may have a structure comprising a source electrode and drain electrode, an organic semiconductor layer which acts as a current channel between them and contains the aforementioned conjugated compound (preferably as an organic thin-film layer, same hereunder), and a gate electrode that controls the level of current flowing through the current channel, and the transistor may be a field-effect type or static induction type, for example.

An organic thin-film field-effect transistor may have a structure comprising a source electrode and drain electrode, an organic semiconductor layer which acts as a current channel between them and contains a conjugated compound, a gate electrode that controls the level of current flowing through the current channel, and an insulating layer situated between the organic semiconductor layer and the gate electrode. Most preferably, the source electrode and drain electrode are provided in contact with the organic semiconductor layer containing the conjugated compound, and the gate electrode is provided sandwiching the insulating layer which is also in contact with the organic semiconductor layer.

A static induction-type organic thin-film transistor preferably has a source electrode and drain electrode, an organic semiconductor layer which acts as a current channel between them and contains a conjugated compound, and a gate electrode that controls the level of current flowing through the current channel, preferably with the gate electrode in the organic semiconductor layer. Most preferably, the source electrode, the drain electrode and the gate electrode formed in the organic semiconductor layer are provided in contact with the organic semiconductor layer containing the conjugated compound. The structure of the gate electrode may be any one that forms a current channel for flow from the source electrode to the drain electrode, and that allows the level of current flowing through the current channel to be controlled by the voltage applied to the gate electrode; an example of such a structure is a combshaped electrode.

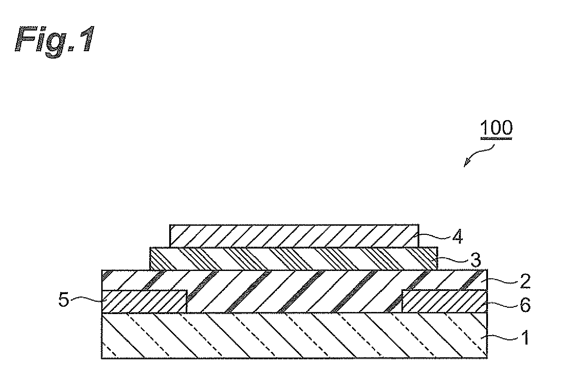

Fig. 1 is a schematic cross-sectional view of an organic thin-film transistor (organic thin-film field-effect transistor) according to a first embodiment. The organic thin-film transistor 100 shown in Fig. 1 comprises a substrate 1, a source electrode 5 and drain electrode 6 formed at a fixed spacing on the substrate 1, an organic semiconductor layer 2 formed on the substrate 1 covering the source electrode 5 and drain electrode 6, an insulating layer 3 formed on the organic semiconductor layer 2, and a gate electrode 4 formed on the insulating layer 3 covering the region of the insulating layer 3 between the source electrode 5 and drain electrode 6.

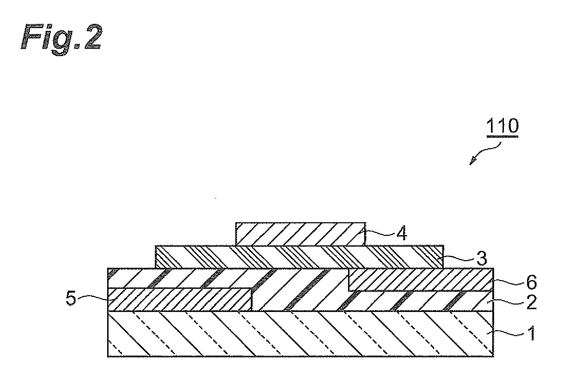

Fig. 2 is a schematic cross-sectional view of an organic thin-film transistor (organic thin-film field-effect transistor) according to a second embodiment. The organic thin-film transistor 110 shown in Fig. 2 comprises a substrate 1, a source electrode 5 formed on the substrate 1, an organic semiconductor layer 2 formed on the substrate 1 covering the source electrode 5, a drain electrode 6 formed on the organic semiconductor layer 2 at a prescribed spacing from the source electrode. 5, an insulating layer 3 formed on the organic semiconductor layer 2 and drain electrode 6, and a gate electrode 4 formed on the insulating layer 3 covering the region of the insulating layer 3 between the source electrode 5 and drain electrode 6.

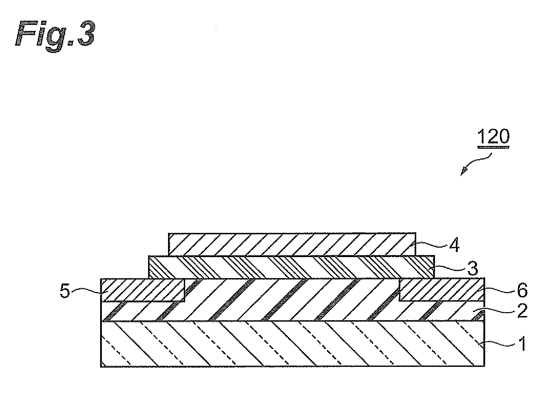

Fig. 3 is a schematic cross-sectional view of an organic thin-film transistor (organic thin-film field-effect transistor) according to a third embodiment. The organic thin-film transistor 120 shown in Fig. 3 comprises a substrate 1, an organic semiconductor layer 2 formed on the substrate 1, a source electrode 5 and drain electrode 6 formed at a prescribed spacing on the organic semiconductor layer 2, an insulating layer 3 formed on the organic semiconductor layer 2 covering the source electrode 5 and drain electrode 6, and a gate electrode 4 formed on the insulating layer 3, covering a portion of the region of the insulating layer 3 under which the source electrode 5 is formed and a portion of the region of the insulating layer 3 under which the drain electrode 6 is formed.

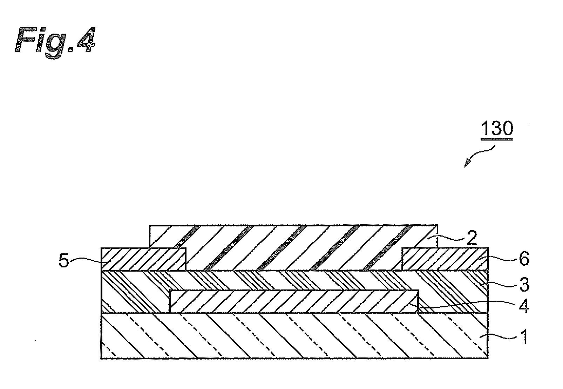

Fig. 4 is a schematic cross-sectional view of an organic thin-film transistor (organic thin-film field-effect transistor) according to a fourth embodiment. The organic thin-film transistor 130 shown in Fig. 4 comprises a substrate 1, a gate electrode 4 formed on the substrate 1, an insulating layer 3 formed on the substrate 1 covering the gate electrode 4, a source electrode 5 and drain electrode 6 formed at a prescribed spacing on the insulating layer 3 covering portions of the region of the insulating layer 3 under which the gate electrode 4 is formed, and an organic semiconductor layer 2 formed on the insulating layer 3 covering portions of the source electrode 5 and drain electrode 6.

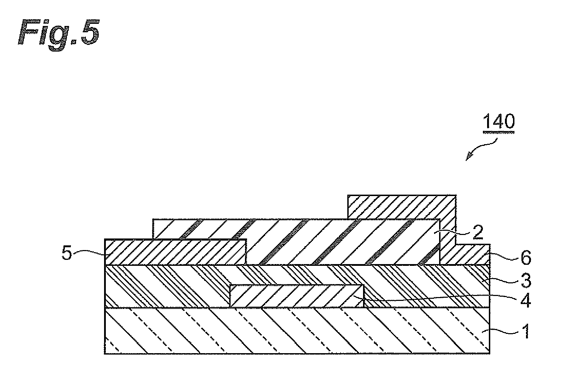

Fig. 5 is a schematic cross-sectional view of an organic thin-film transistor (organic thin-film field-effect transistor) according to a fifth embodiment. The organic thin-film transistor 140 shown in Fig. 5 comprises a substrate 1, a gate electrode 4 formed on the substrate 1, an insulating layer 3 formed on the substrate 1 covering the gate electrode 4, a source electrode 5 formed on the insulating layer 3 covering a portion of the region of the insulating layer 3 under which the gate electrode 4 is formed, an organic semiconductor layer 2 formed on the insulating layer 3 covering a portion of the source electrode 5, and a drain electrode 6 formed on the insulating layer 3 at a prescribed spacing from the source electrode 5 and covering a portion of the region of the organic semiconductor layer 2.

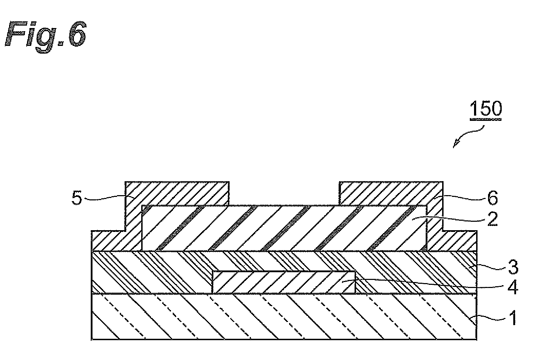

Fig. 6 is a schematic cross-sectional view of an organic thin-film transistor (organic thin-film field-effect transistor) according to a sixth embodiment. The organic thin-film transistor 150 shown in Fig. 6 comprises a substrate 1, a gate electrode 4 formed on the substrate 1, an insulating layer 3 formed on the substrate 1 covering the gate electrode 4, an organic semiconductor layer 2 formed covering the region of the insulating layer 3 under which the gate electrode 4 is formed, a source electrode 5 formed on the insulating layer 3 covering a portion of the region of the organic semiconductor layer 2, and a drain electrode 6 formed on the insulating layer 3 at a prescribed spacing from the source electrode 5 and covering a portion of the region of the organic semiconductor layer 2.

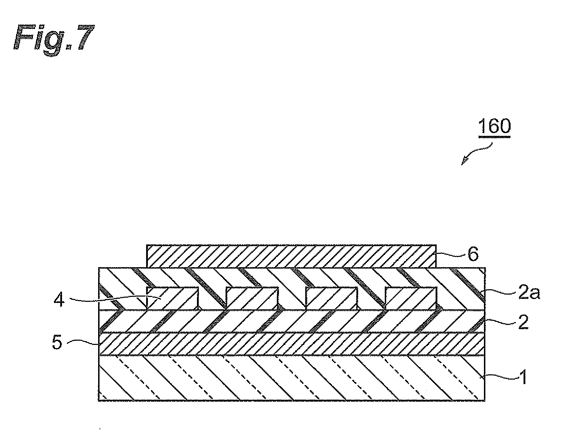

Fig. 7 is a schematic cross-sectional view of an organic thin-film transistor (static induction-type organic thin-film transistor) according to a seventh embodiment. The organic thin-film transistor 160 shown in Fig. 7 comprises a substrate 1, a source electrode 5 formed on the substrate 1, an organic semiconductor layer 2 formed on the source electrode 5, a plurality of gate electrodes 4 formed at prescribed spacings on the organic semiconductor layer 2, an organic semiconductor layer 2a formed on the organic semiconductor layer 2 covering all of the gate electrodes 4, (the material composing the organic semiconductor layer 2a may be the same as or different from that of the organic semiconductor layer 2) and a drain electrode 6 formed on the organic semiconductor layer 2a.

In the organic thin-film transistors of the first to seventh embodiments, the organic semiconductor layer 2 and/or the organic semiconductor layer 2a contains a preferred conjugated compound described above and forms a current channel between the source electrode 5 and drain electrode 6. The gate electrode 4 controls the level of current flowing through the current channel of the organic semiconductor layer 2 and/or organic semiconductor layer 2a by application of voltage.

This type of organic thin-film field-effect transistor can be manufactured by a publicly known process, such as the process described in Japanese Unexamined Patent Application Publication HEI No. 5-110069, for example. A static induction-type organic thin-film transistor can be manufactured by a publicly known process, such as the process described in Japanese Unexamined Patent Application Publication No. 2004-006476, for example.

The material of the substrate 1 may be any one that does not inhibit the characteristics of the organic thin-film transistor. The substrate 1 used may be a glass panel, flexible film substrate or plastic panel.

Although organic solvent-soluble conjugated compounds are highly advantageous in terms of production and preferred for forming the organic semiconductor layer 2, the conjugated compounds mentioned above have excellent solubility and thus allow satisfactory formation of an organic thin-film comprising the organic semiconductor layer 2 by the method for producing an organic thin-film described above.

The insulating layer 3 in contact with the organic semiconductor layer 2 may be any material with high electrical insulating properties, and any publicly known one may be used. As examples there may be mentioned SiOx, SiNx, Ta₂O₅, polyimide, polyvinyl alcohol, polyvinylphenol, organic glass and photoresists. From the viewpoint of low voltage, it is preferred to use a material with high permittivity for the insulating layer 3.

When the organic semiconductor layer 2 is to be formed on the insulating layer 3, the organic semiconductor layer 2 may be formed after surface modification by treatment of the surface of the insulating layer 3 with a surface treatment agent such as a silane coupling agent in order to improve the interfacial properties between the insulating layer 3 and organic semiconductor layer 2. Surface treatment agents include long-chain alkylchlorosilanes, long-chain alkylalkoxysilanes, fluorinated alkylehlorosilanes, fluorinated alkylalkoxysilanes and silylamine compounds such as hexamethyldisilazane. Before treatment with the surface treatment agent, the insulating layer surface may be pre-treated by ozone UV or O₂ plasma.

After the organic thin-film transistor has been fabricated, a protecting film is preferably formed on the organic thin-film transistor to protect the element. This will help prevent reduction in the characteristics of the organic thin-film transistor when the organic thin-film transistor has been blocked from air. A protecting film can also minimize adverse external effects during the step of forming an operating display device on the organic thin-film transistor.

The method of forming the protecting film may involve covering with a UV curing resin, thermosetting resin or inorganic SiONx film. For effective shielding from air, the steps after fabrication of the organic thin-film transistor and before formation of the protecting film are preferably carried out without exposure to air (for example, in a dry nitrogen atmosphere or in a vacuum).

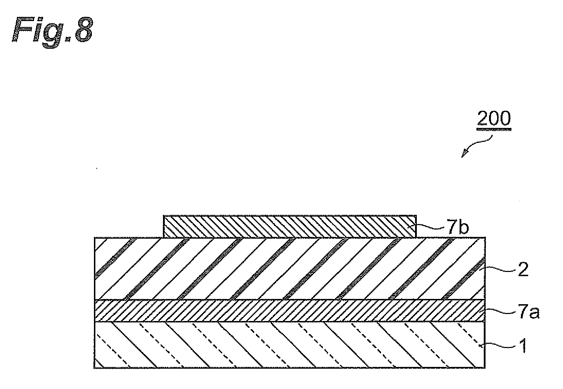

Application of an organic thin-film of the invention in a photoelectric conversion element will now be explained. A solar cell or optical sensor are typical photoelectric conversion elements. Fig. 8 is a schematic cross-sectional view of a solar cell according to an embodiment of the invention. The solar cell 200 shown in Fig. 8 comprises a substrate 1, a first electrode 7a formed on the substrate 1, an organic semiconductor layer 2 made of an organic thin-film that contains a conjugated compound formed on the first electrode 7a, and a second electrode 7b formed on the organic semiconductor layer 2.

In this solar cell 200, a transparent or semi-transparent electrode is used for either or both the first electrode 7a and second electrode 7b. As electrode materials there may be used metals such as aluminum, gold, silver, copper, alkali metal and alkaline earth metals or their semi-transparent films, or transparent conductive films. In order to obtain high open voltage, it is preferred to select the electrodes so as to produce a large work function difference. Charge generators, sensitizing agents and the like may also be added in order to increase photosensitivity in the organic semiconductor layer 2. The substrate 1 may be a silicon substrate, glass panel, plastic panel or the like.

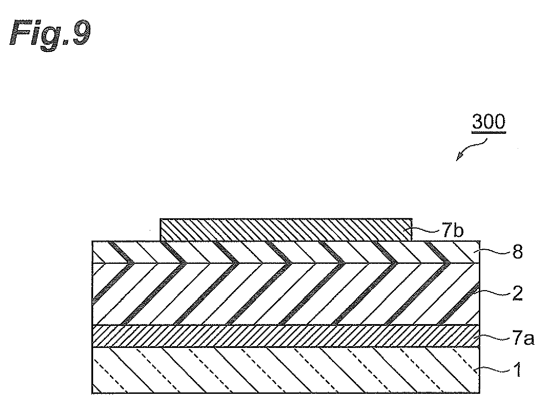

Fig. 9 is a schematic cross-sectional view of an optical sensor according to a first embodiment. The optical sensor 300 shown in Fig. 9 comprises a substrate 1, a first electrode 7a formed on the substrate 1, an organic semiconductor layer 2 made of an organic thin-film comprising a conjugated compound, formed on the first electrode 7a, a charge generation layer 8 formed on the organic semiconductor layer 2, and a second electrode 7b formed on the charge generation layer 8.

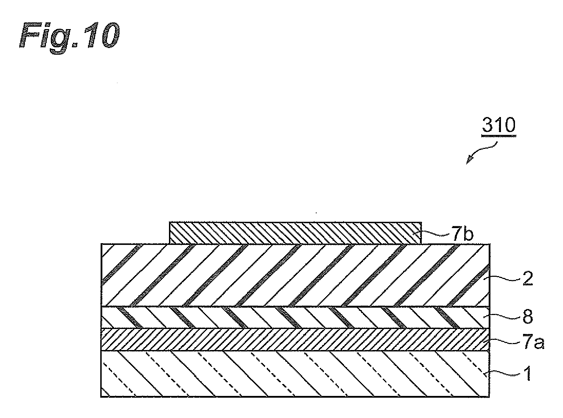

Fig. 10 is a schematic cross-sectional view of an optical sensor according to a second embodiment. The optical sensor 310 shown in Fig. 10 comprises a substrate 1, a first electrode 7a formed on the substrate 1, a charge generation layer 8 formed on the first electrode 7a, an organic semiconductor layer 2 made of an organic thin-film comprising a conjugated compound, formed on the charge generation layer 8, and a second electrode 7b formed on the organic semiconductor layer 2.

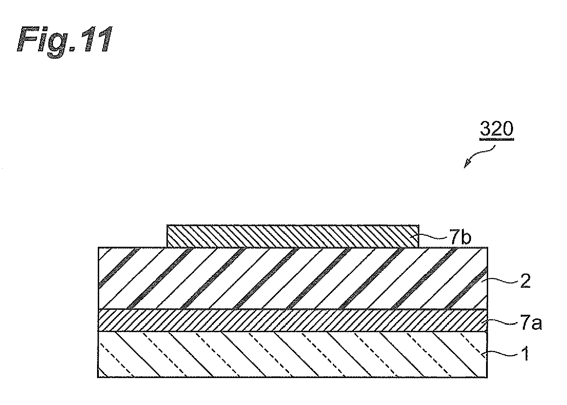

Fig. 11 is a schematic cross-sectional view of an optical sensor according to a third embodiment. The optical sensor 320 shown in Fig. 11 comprises a substrate 1, a first electrode 7a formed on the substrate 1, an organic semiconductor layer 2 made of an organic thin-film that contains a conjugated compound formed on the first electrode 7a, and a second electrode 7b formed on the organic semiconductor layer 2.

In the optical sensors of the first to third embodiments, a transparent or semi-transparent electrode is used for either or both the first electrode 7a and second electrode 7b. The charge generation layer 8 is a layer that generates an electrical charge upon absorption of light. As electrode materials there may be used metals such as aluminum, gold, silver, copper, alkali metal and alkaline earth metals or their semi-transparent films, or transparent conductive films. Carrier generators, sensitizing agents and the like may also be added in order to increase photosensitivity in the organic semiconductor layer 2. The substrate 1 may be a silicon substrate, glass panel, plastic panel or the like.

### Examples

The present invention will now be explained in detail by examples, with the understanding that the invention is not limited to the examples.

### [Measuring conditions]

The nuclear magnetic resonance (NMR) spectra were measured using a JMN-270 (270 MHz for ¹H measurement) or a JMNLA-600 (600 MHz for ¹⁹F measurement), both trade names of JEOL Corp. The chemical shifts are represented as parts per million (ppm). Tetramethylsilane (TMS) was used as the internal standard (0 ppm). The coupling constant (J) is represented in Hz, and the symbols s, d, t, q, m and br respectively represent singlet, doublet, triplet, quartet, multiplet and broad.

The mass spectrometry (MS) was performed using a GCMS-QP5050A, trade name of Shimadzu Corp., by electron ionization (EI) or direct inlet (DI). The silica gel used for separation by column chromatography was Silicagel 60N (40-50 µm), trade name of Kanto Kagaku Co., Ltd. All of the chemical substances were reagent grade and purchased from Wako Pure Chemical Industries, Ltd., Tokyo Kasei Kogyo Co., Ltd., Kanto Kagaku Co., Ltd., Nacalai Tesque, Inc., Sigma Aldrich Japan, KK. or Daikin Chemicals Co., Ltd.

Cyclic voltammetry (CV) was performed using a CV-50W, trade name of BAS, Inc. as the measuring apparatus, with a Pt electrode by BAS, Inc. as the work electrode, Pt wire as the counter electrode and Ag wire as the reference electrode. The sweep rate during measurement was 100 mV/sec, and the scanning potential range was -2.0 V to 1.6 V. The reduction potential and oxidation potential were measured after completely dissolving 1 × 10⁻³ mol/L of the conjugated compound and 0.1 mol/L of tetrabutylammonium hexafluorophosphate (TBAPF6) as a supporting electrolyte in a monofluorobenzene solvent.

The reaction under microwave irradiation was conducted using an Initiator^{™} Ver.2.5 by Biotage, with an output of 400W, 2, 45 GHz.

### (Synthesis of starting compounds)

Following scheme 1 shown below, compound A represented by formula (23 a) was used as starting material for synthesis of compound D represented by formula (25) and compound E represented by formula (26b), as the starting compounds for the conjugated compound, via compound B represented by formula (23b) and compound C represented by formula (24). This will now be explained in detail.

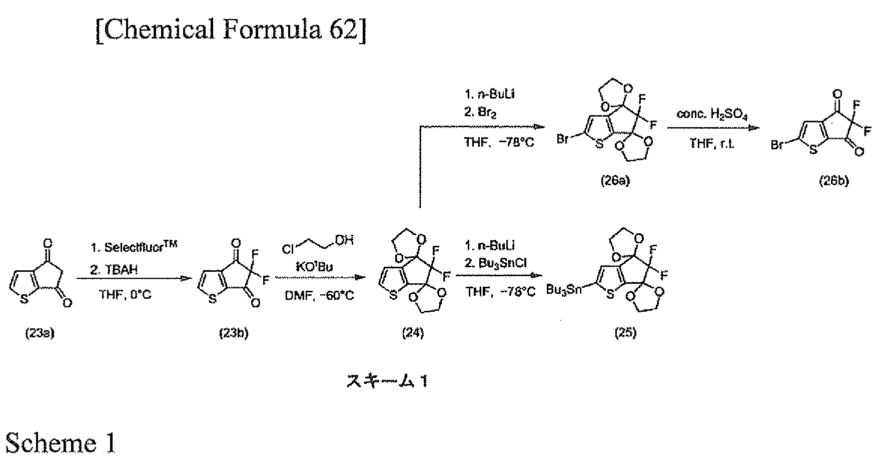

### <Synthesis of Compound B>

Compound A represented by formula (23a) was synthesized by a method described in the literature (J. Chem. Soc. Perkin Trans, 1. Organic and Bio-Organic Chemistry 1992, 21, 2985-2988). Next, compound A (1.00 g, 6.58 mol) and the fluorinating agent Selectfluor^{™} (registered trademark) (5.60 g, 15.8 mol) were placed in a 300 mL three-necked flask and THF (65 mL) was added to dissolve them. Tetrabutylammonium hydroxide (TBAH) (10% methanol solution) (3.76 g, 14.5 mol) was then added and the mixture was stirred at 0°C for 12 hours. The solvent was distilled off under reduced pressure, and then water was added, the aqueous layer was extracted with ethyl acetate, and the organic layer was dried over magnesium sulfate and concentrated under reduced pressure. The obtained concentrate was purified by silica gel column chromatography (hexane/ethyl acetate = 3/1 (volume ratio)) to obtain compound B represented by formula (23b) (0.934 g, 75% yield) as a light yellow solid.

The evaluation results for the obtained compound B are as follows.
mp 156-158°C; TLC R_{f} = 0.29 (hexane/ethyl acetate = 2/1 (volume ratio)); ¹H-NMR (400 MHz, CDCl₃) δ 7.60 (d, 1H, J = 4.8 Hz), 8.28 (d, 1H, J = 4.8 Hz); MS (EI) m/z = 188 (M⁺)

### <Synthesis of Compound C>

Compound B (1.97 g, 10.48 mmol) was placed in a 200 mL three-necked flask, N,N'-dimethylformamide (DMF) (50 mL) was added to dissolve it, and then 2-chloroethanol (3.37 g, 41.91 mmol) was further added. Potassium tert-butoxide dissolved in DMF (50 mL) was then added dropwise thereto at -60°C. Upon completion of the dropwise addition, the mixture was stirred at room temperature for 4 hours, and water was added to suspend the reaction. The aqueous layer was extracted with ethyl acetate and rinsed with water, and then the organic layer was dried over magnesium sulfate, filtered and concentrated under reduced pressure. The obtained concentrate was purified by silica gel column chromatography (hexane/ethyl acetate = 3/1 (volume ratio)) to obtain compound C represented by formula (24) (1.58g,55% yield) as a white solid.

The evaluation results for the obtained compound C are as follows.
mp 117-122°C; TLC R_{f} = 0.34 (hexane/ethyl acetate = 2/1 (volume ratio)); ¹H-NMR (400 MHz, CDCl₃) δ 4.26 (s, 8H), 7.02 (d, 1H, J = 4.8 Hz), 7.51 (d, 1H, J = 5.1 Hz); MS (EI) m/z = 276 (M⁺)

### <Synthesis of Compound D>

Compound C (500 mg, 1.81 mmol) was placed in a 50 mL three-necked flask, and THF (18 mL) was added to dissolve it. Next, n-butyllithium (1.58 M hexane solution, 2.29 mL, 3.62 mmol) was added thereto at -78°C. After stirring for 0.5 hour, tributyltin chloride (1.09 mL, 3.98 mmol) was added and the temperature was slowly raised to room temperature. After 1 hour, water was added to suspend the reaction. The aqueous layer was extracted with ethyl acetate and rinsed with water, and then the organic layer was dried over magnesium sulfate, filtered and concentrated under reduced pressure. The obtained concentrate was purified by alumina-column chromatography (hexane/ethyl acetate = 10/1 (volume ratio)) to obtain compound D represented by formula (25) (1.02 g, 99% yield) as a colorless liquid.

The evaluation results for the obtained compound D are as follows.
TLC R_{f} = 0.30(hexane); ¹H-NMR (400 MHz, CDCl₃) δ 0.89 (t, 9H, J = 7.2 Hz), 1.08-1.13 (m, 6H), 1.24-1.38 (m, 6H), 1.49-1.60 (m, 6H), 4.23-4.28 (m, 8H), 7.03 (s, 1H); MS (EI) m/z = 566 (M⁺)

### <Synthesis of Compound E>

Compound C (1.00 g, 3.62 mmol) was placed in a 100 mL three-necked flask, and THF (30 mL) was added to dissolve it. Next, n-butyllithium (1.58 M hexane solution, 2.75 mL, 4.34 mmol) was added thereto at -78C. After stirring for 0.5 hour, bromine (0.29 mL, 5.43 mmol) was added and the temperature was slowly raised to room temperature. After 1 hour, water was added to suspend the reaction. The aqueous layer was extracted with ethyl acetate and rinsed with saturated aqueous sodium thiosulfate, and after further rinsing with water, the organic layer was dried over magnesium sulfate. The solvent was distilled off under reduced pressure, and the crude product was passed through silica gel column chromatography (hexane/ethyl acetate = 3/1 (volume ratio)) to obtain a crude product of the intermediate compound represented by formula (26a) above.

The obtained intermediate compound was placed in a 100 mL volumetric flask and dissolved in THF (30 mL). Concentrated sulfuric acid (30 mL) was added and the mixture was stirred at room temperature for 12 hours. The reaction mixture was poured into ice and extracted with water. The organic layer was rinsed with aqueous saturated sodium hydrogencarbonate and water in that order and dried over magnesium sulfate, and then filtered and concentrated under reduced pressure. The obtained concentrate was purified by silica gel column chromatography (ethyl acetate) to obtain compound E represented by formula (26b) (877 mg, 91 % yield in 2 steps) as a brown solid.

The evaluation results for the obtained compound E are as follows.
TLC R_{f} = 0.21 (hexane/ethyl acetate = 3/1 (volume ratio)); ¹H-NMR (400 MHz, CDCl₃) δ 7.60 (s, 1H); MS (EI) m/z = 266 (M⁺)

(Synthesis of synthetic intermediate for conjugated compound 1) Compound H represented by formula (32), as a synthetic intermediate for a conjugated compound, was synthesized via compound F represented by formula (30) and compound G represented by formula (31). This will now be explained in detail.

### <Synthesis of Compound F>

After placing 2,5-dibromothiophene (48 mg, 0.199 mmol), 5-tributyl-3-hexylthiophene (200 mg, 0.437 mmol) and tetrakis(triphenylphosphine)palladium(0) (11 mg, 0.0199 mmol) in a heat-dried stoppered test tube, toluene (2 mL) was added to dissolve them. After stirring the mixture at 120°C for 12 hours, it was allowed to cool at room temperature. The solvent was then distilled off under reduced pressure, and the obtained crude product was purified by silica gel column chromatography (hexane) to obtain compound F represented by formula (30) (48 mg, 58% yield) as a yellow liquid.

The evaluation results for the obtained compound F are as follows.
TLC R_{f} = 0.75(hexane); ¹HNMR (400 MHz, CDCl₃) δ 0.89 (m, 6H), 1.22-1.44 (m, 12H), 1.50-1.72 (m, 4H), 2.58 (t, 4H, J = 7.8 Hz), 6.80 (s, 2H), 7.00 (s, 2H), 7.03 (s, 2H); MS (EI) m/z = 416 (M⁺).

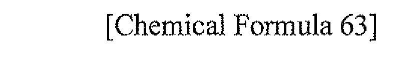

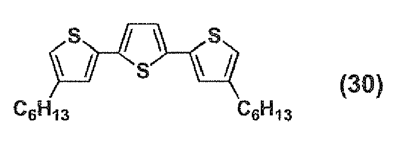

### <Synthesis of Compound G>

After placing compound F (100 mg, 0.240 mmol) and tetramethylethylenediamine (58 mg, 0.504 mmol) in a heat-dried 30 mL two-necked flask, diethyl ether (3 mL) was added to dissolve them. There was slowly added thereto n-butyllithium (1.58 M hexane solution, 0.319 mL, 0.504 mmol) at 0°C. After stirring for 2 hours, tributyltin chloride (0.221 ml, 0.814 mmol) was slowly added at -78°C and the temperature was gradually raised to room temperature. Water was added to suspend the reaction, the aqueous layer was extracted using diethyl ether, and the organic layer was rinsed with aqueous saturated copper sulfate and then dried over magnesium sulfate. The solvent was then distilled off under reduced pressure, and the obtained crude product was purified by alumina column chromatography (hexane) to obtain compound G represented by formula (31) (165 mg, 69% yield) as a yellow liquid.

The evaluation results for the obtained compound G are as follows.
TLC R_{f} = 1.0 (hexane); ¹H NMR (400 MHz, CDCl₃) δ 0.84-0.96 (m, 24H), 1.05-1.20 (m, 12H), 1.25-1.45 (m, 24H), 1.50-1.70 (m, 16H), 2.51 (t, 4H, J = 8.0 Hz), 7.02 (s, 2H), 7.14 (s, 2H).

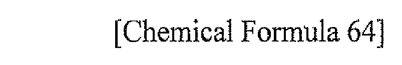

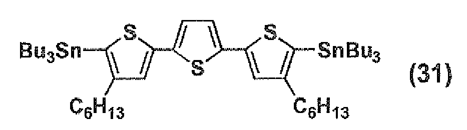

### <Synthesis of Compound H>

After placing compound F (50 mg, 0.050 mmol), compound E (29 mg, 0.11 mmol) and tetrakis(triphenylphosphine)palladium(0) (6 mg, 0.0050 mmol) in a test tube, toluene (1 mL) was added to dissolve them. After stirring the mixture at 120°C for 12 hours, it was allowed to cool at room temperature. The solvent was distilled off under reduced pressure, and the obtained crude product was passed through silica gel column chromatography (CHCl₃) and then purified by GPC (CHCl₃) to obtain compound H represented by formula (32) (16 mg, 49% yield) as a red solid.

The evaluation results for the obtained compound H are as follows.
TLC R_{f} = 0.48 (hexane/ethyl acetate = 3/1 (volume ratio)); ¹H NMR (400 MHz, CDCl₃) δ 0.92 (t, 6H, J = 7.1 Hz), 1.29-1.50 (m, 12H), 1.69-1.75 (m, 4H), 2.84 (t, 4H, J = 7.8 Hz), 7.14 (s, 2H), 7.23 (s, 2H), 7.48 (s, 2H); MS (EI) m/z = 788 (M⁺).

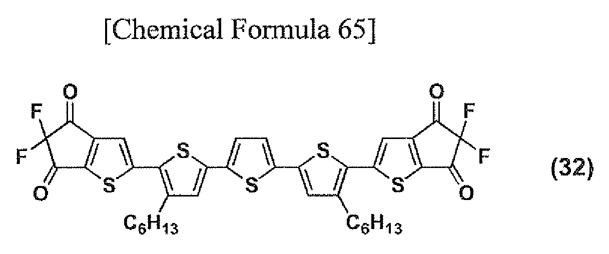

### (Example 1)

### <Synthesis of conjugated compound I>

A solution of compound H (6 mg, 0.0127 mmol), malononitrile (15 mg, 0.227 mmol) and ammonium acetate (2 mg, 0.0254 mmol) in absolute ethanol (1 mL) was circulated in a 20 mL volumetric flask at 80°C for 30 minutes. After cooling to room temperature, water was added and the solution was acidified with concentrated hydrochloric acid. The produced solid was subjected to suction filtration and washed with water. The obtained solid was purified by GPC (CHCl₃) to obtain conjugated compound I represented by formula (33) (3 mg, 25% yield).

The evaluation results for the obtained conjugated compound I are as follows. Upon CV measurement of the obtained conjugated compound I, a reversible reduction wave was observed at -0.76 V, and a low LUMO level and an excellent electron transport property were confirmed.
TLC R_{f} = 0.55 (CHCl₃); ¹H NMR (400 MHz, CDCl₃) δ 0.88-0.90 (m, 6H), 1.29-1.50 (m, 12H), 1.69-1.79 (m, 4H), 2.85 (t, 4H, J = 8.1 Hz), 7.20 (s, 2H), 7.24-7,28 (m, 2H), 7.78 (s, 2H); MS (MALDI TOF) m/z980 (M⁺).

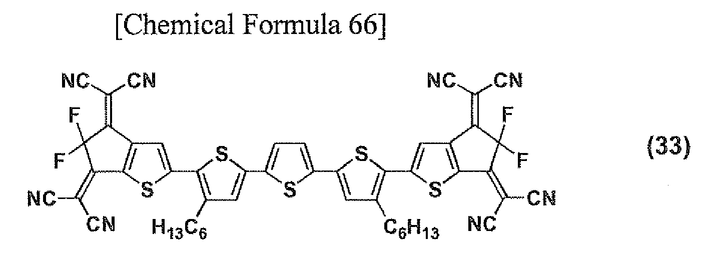

(Synthesis of synthetic intermediate for conjugated compound 2) Compound K represented by formula (35), as a synthetic intermediate for a conjugated compound, was synthesized via compound J represented by formula (34). This will now be explained in detail.

### <Synthesis of Compound J>

After placing 5,5'-dibromo-4,4'-dihexyl-2,2'-bithiophene (492 mg, 1.00 mmol) in a 20 mL three-necked flask, it was dissolved in THF (10 mL). Next, n-butyllithium (1.58 M hexane solution, 1.39 mL, 2.20 mmol) was added thereto at -78°C. After stirring for 1 hour, tributyltin chloride (0.543 ml, 2.00 mmol) was added and the temperature was slowly raised to room temperature. After 2 hours, water and a trace amount of hydrochloric acid were added to suspend the reaction. The aqueous layer was extracted with diethyl ether and rinsed with water, and then the organic layer was dried over magnesium sulfate. The solvent was distilled off under reduced pressure, and then the obtained liquid was purified by GPC (CHCl₃) to obtain compound J represented by formula (34) (630 mg, 69% yield) as a yellow liquid.

The evaluation results for the obtained compound J are as follows.
TLC R_{f} = 1.0(hexane); ¹H NMR (400 MHz, CDCl₃) δ 0.84-0.94 (m, 24H), 1.02-1.20 (m, 12H), 1.26-1.39 (m, 24H), 1.46-1.61 (m, 16H), 2.51 (t, 4H, 8.0 Hz), 7.13 (s, 2H); MS (EI) m/z 912 (M⁺).

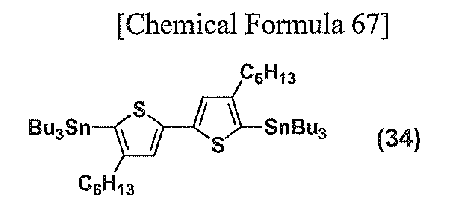

### <Synthesis of Compound K>

After placing compound J (50 mg, 0.055 mmol), compound E (32 mg, 0.12 mmol) and tetrakis(triphenylphosphine)palladium(0) (6 mg, 0.005 mmol) in a stoppered test tube, the mixture was dissolved in toluene (1 mL). After stirring the mixture at 120°C for 12 hours, it was allowed to cool at room temperature. The solvent was then distilled off under reduced pressure, and the obtained crude product was passed through silica-column chromatography (CHCl₃) and then purified by GPC (CHCl₃) to obtain compound K represented by formula (35) (19 mg, 49% yield) as an orange solid. The oxidation potential of compound K was 0.48 V, and the reduction potential was -1.87 V. The peak wavelength in the absorption spectrum was 472 nm.

The evaluation results for the obtained compound K are as follows.
TLC R_{f} = 0.43 (hexane/ethyl acetate = 5/1 (volume ratio)); ¹H NMR (400 MHz, CDCl₃) δ 0.88-0.96 (m, 6H), 1.28-1.49 (m, 12H), 1.65-1.76 (m, 4H), 2.85 (t, 4H, J = 7.9 Hz), 7.19 (s, 2H), 7.51 (s, 2H); MS (EI) m/z 706 (M⁺).

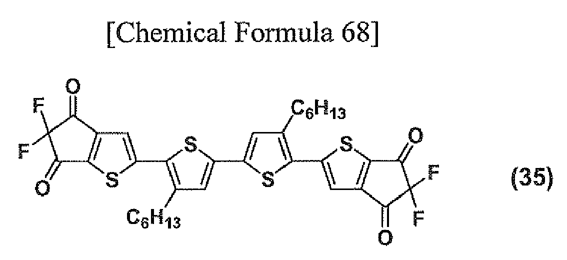

### (Example 2)

### <Synthesis of conjugated compound L>

A solution of compound K (30 mg, 0.0425 mmol), malononitrile (17 mg, 0.255 mmol) and ammonium acetate (7 mg, 0.0850 mmol) in absolute ethanol (1 mL) was circulated in a 20 mL volumetric flask at 80°C for 30 minutes. After cooling to room temperature, water was added and the solution was acidified with concentrated hydrochloric acid. The produced solid was subjected to suction filtration and washed with water. The obtained solid was purified by GPC (CHCl₃) to obtain conjugated compound L represented by formula (36) (11 mg, 29% yield).

The evaluation results for the obtained conjugated compound L are as follows. Upon CV measurement of the obtained conjugated compound L, a reversible reduction wave was observed at -0.67 V, and a low LUMO level and an excellent electron transport property were confirmed.
TLC *R_{f}* = 0.69 (CHCl₃)
¹H NMR (400 MHz, CDCl₃) δ 0.80-0.96 (m, 6H), 1.25-1.44 (m, 12H), 1.65-1.80 (m, 4H), 2.88 (t, 4H, J = 8.0 Hz), 6.80 (s, 2H), 7.21-7.28 (m, 2H), 7.87 (s, 2H)
MS (MALDI TOF) m/z 898 (M⁺).

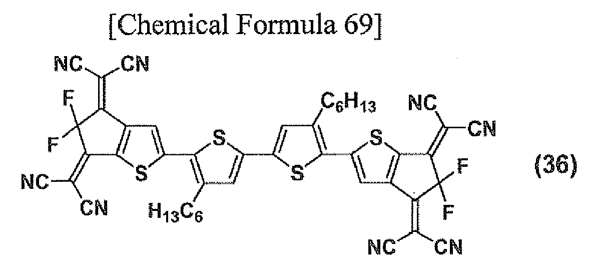

### (Example 3)

### <Fabrication of organic thin-film transistor and evaluation of transistor property>

A thermal oxidation film (silicon oxide film)-attached low resistance silicon wafer (gate electrode/insulating layer) was dipped in ethanol, distilled water and acetone in that order, and ultrasonic cleaning was performed. The silicon wafer was then subjected to UV-ozone cleaning to obtain a substrate with a hydrophilic surface. The substrate was dipped in hexamethyldisilazane:chloroform at room temperature and subjected to ultrasonic cleaning with chloroform to obtain a surface-treated substrate.

Next, a coating solution was prepared comprising conjugated compound I synthesized in Example 1 dissolved in chloroform. The solution was formed into a film by spin coating on a surface-treated substrate, to form an organic thin-film. Gold electrodes (source electrode, drain electrode) were formed on the organic thin-film by vacuum vapor deposition using a metal mask, to fabricate an organic thin-film transistor.

The obtained organic thin-film transistor was measured for organic transistor properties using a Semiconductor Parameter Analyzer (trade name "4200-SCS", by Keithley Instruments, Inc.), while varying the gate voltage Vg and source-drain voltage Vsd, and satisfactory n-type semiconductor Id-Vg characteristics were obtained. This indicated that the conjugated compound I has an excellent electron transport property.

### (Example 4)

### <Synthesis of conjugated compound M>

Compound E (1.00 g, 3.74 mmol), malononitrile (989 mg, 14.97 mmol), ammonium acetate (288 mg, 3.74 mmol), toluene (40 mL) and acetic acid (4 mL) were added to a 100 mL volumetric flask and circulated for 4 hours. The mixture was washed with water and extracted with ethyl acetate. The organic layer was dried over sodium sulfate, the solvent was distilled off, and the residue was vacuum dried. It was then purified with a silica gel column (hexane/ethyl acetate = 3/1 (volume ratio)) to obtain conjugated compound M represented by formula (37) as a yellow solid (531 mg, 39% yield).

The evaluation results for the obtained conjugated compound M are as follows.
TLC R_{f} = 0.4 (hexane/ethyl acetate = 3/1 (volume ratio))
¹H NMR (400 MHz, CDCl₃) δ 7.96 (s, 1H)
GC-MS (DI) m/z = 3 62 (M⁺)

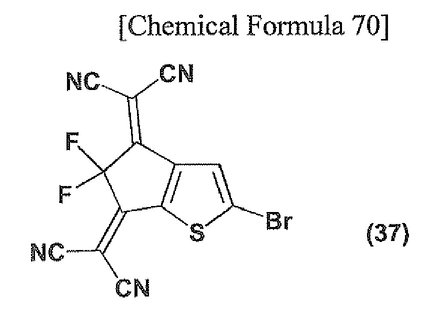

### <Synthesis of conjugated compound N>

After placing conjugated compound M (121 mg, 0.33 mmol), compound J (100 mg, 0.15 mmol), tris(dibenzylideneacetone)dipalladium (Pd₂(dba)₃) (3 mg, 0.003 mmol), tri-*o*-tolylphosphine (4 mg, 0.013 mmol) and chlorobenzene (3 mL) in a 5 mL test tube, reaction was conducted with microwaves (180°C, 5 min). Purification was performed with a silica gel column (chloroform) and GPC, to obtain conjugated compound N represented by formula (38) as a dark violet solid (113 mg, 83% yield).

The evaluation results for the obtained conjugated compound N are as follows. Upon CV measurement of the obtained conjugated compound N, a reversible reduction wave was observed at -0.67 V, and a low LUMO level and an excellent electron transport property were confirmed. The peak wavelength in the absorption spectrum of the obtained conjugated compound N was 591 nm.
¹H NMR (400 MHz, CDCl₃) δ 0.90 (t, 3H, J = 6.9 Hz), 1.34 (m, 8H), 1.47 (m, 4H), 1.75 (m, 4H), 2.88 (t, 4H, J = 7.8 Hz), 7.26 (s, 2H), 7.86 (s, 2H)
MALDI TOFMS: m/z = 898

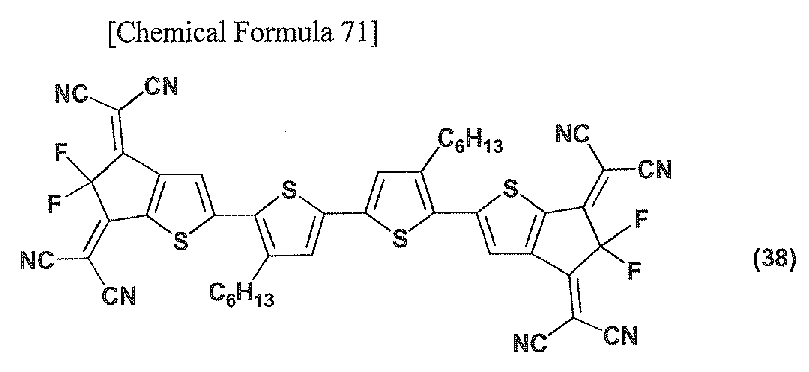

### (Synthesis of synthetic intermediate for conjugated compound 3)

### <Synthesis of Compound P>

After placing Compound O represented by formula (39) (1.90 g, 5.49 mmol), synthesized with reference to Macromolecules (2003), 36(8), 2705-2711, in a 100 mL 2-necked flask, the interior of the flask was substituted with nitrogen and THF (55 mL) was added. After cooling to -78°C, tetramethylethylenediamine (2 mL, 13.72 mmol) and n-BuLi (6.4 mL, 13.72 mmol) were added and reaction was conducted. After 1 hour, tributyltin chloride (3.7 mL, 13.37 mmol) was added at -78°C and the temperature was raised to room temperature. After another hour, water was added and extraction was performed with hexane. The organic layer was dried over sodium sulfate, the solvent was distilled off, and the residue was vacuum dried. Purification was performed with an alumina column (hexane) and GPC, to obtain compound P represented by formula (40) as a yellow liquid (2.70 g, 53% yield). The analysis results and chemical formula for the obtained compound P are shown below.
¹H NMR (400 MHz, CDCl₃) δ 0.80 (m), 0.90 (m), 1.24 (m), 1.35 (m), 1.57 (m), 1.80 (m), 6.89 (s, 2H)

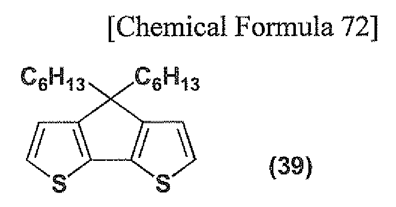

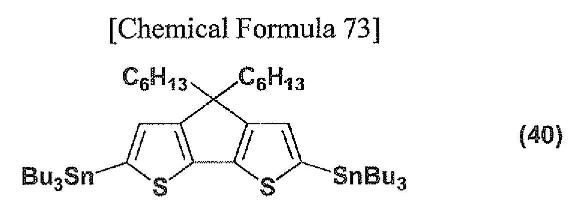

### (Example 5)

### <Synthesis of conjugated compound Q>

After placing compound M(130 mg, 0.36 mmol), compound P (150 mg, 0.16 mmol), Pd₂(dba)₃ (3 mg, 0.003 mmol), tri-o-tolylphosphine (4 mg, 0.013 mmol) and chlorobenzene (3 mL) in a 5 mL test tube, reaction was conducted under microwave irradiation (180°C, 5 min). Purification was performed with a silica gel column (chloroform) and GPC, to obtain conjugated compound Q represented by formula (41) as a dark blueish-black solid (132 mg, 90% yield).

The evaluation results for the obtained conjugated compound Q are as follows. Upon CV measurement of the obtained conjugated compound Q, a reversible reduction wave was observed at -0.65 V, and a low LUMO level and an excellent electron transport property were confirmed. The peak wavelength in the absorption spectrum of the obtained conjugated compound Q was 682 nm.
¹H NMR (400 MHz, CDCl₃) δ 0.82 (t, 3H, J = 6.9 Hz), 0.97 (m), 1.19 (m), 1.98 (t, 4H, J = 7.8 Hz), 7.49 (s, 2H), 7.82 (s, 2H)
MALDI TOFMS: m/z = 910

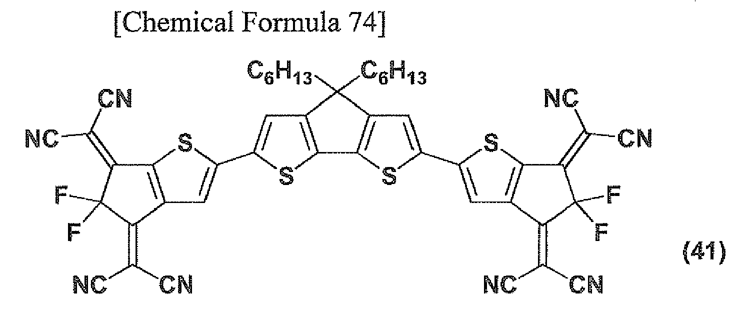

### (Synthesis of synthetic intermediate for conjugated compound 4)

### <Synthesis of Compound S>

After placing Compound R represented by formula (42) (549 mg, 1.00 mmol) in a 100 mL 2-necked flask, the interior of the flask was substituted with nitrogen and THF (15 mL) was added. After cooling to -78°C, tetramethylethylenediamine (0.3 mL), 2.00 mmol) and n-BuLi (1.4 mL, 2.2 mmol) were added and reaction was conducted. After 1 hour, tributyltin chloride (0.6 mL, 2.20 mmol) was added at -78°C and the temperature was raised to room temperature. After another hour, water was added and extraction was performer with hexane. The organic layer was dried over sodium sulfate, the solvent was distilled off, and the residue was vacuum dried. Purification was performed with an alumina column (hexane) and GPC, to obtain compound S represented by formula (43) as a yellow liquid (920 mg, 95% yield). The analysis results and chemical formula for the obtained compound S are shown below.
¹H NMR (400 MHz, CDCl₃) δ 0.81 (m), 0.89 (m), 1.09 (m), 1.35 (m), 1.58 (m), 1.93 (m), 7.38 (s, 1H), 7.39 (d, 2H, 7.2 Hz), 7.63 (d, 2H, 7.2 Hz)

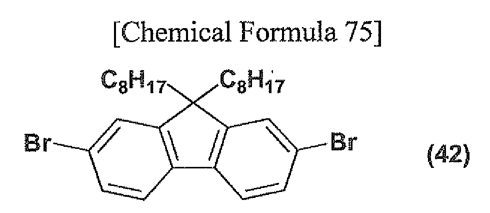

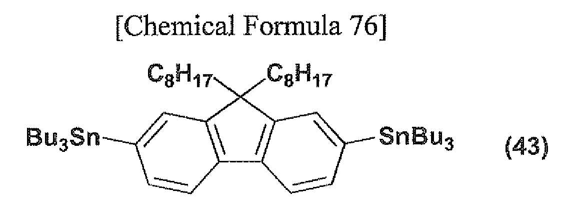

### (Example 6)

### <Synthesis of conjugated compound T>

After placing compound M (124 mg, 0.34 mmol), compound S (150 mg, 0.15 mmol), Pd₂(dba)₃ (3 mg, 0.003 mmol), tri-o-tolylphosphine (4 mg, 0.013 mmol) and chlorobenzene (3 mL) in a 5 mL test tube, reaction was conducted under microwave irradiation (180°C, 5 min). Purification was performed with a silica gel column (chloroform) and GPC, to obtain conjugated compound T represented by formula (44) as a dark reddish-violet solid (130 mg, 88% yield).

The evaluation results for the obtained conjugated compound T are as follows. Upon CV measurement of the obtained conjugated compound T, a reversible reduction wave was observed at -0.65 V, and a low LUMO level and an excellent electron transport property were confirmed.
¹H NMR (400 MHz, CDCl₃) δ 0.58 (m), 0.78 (t, 6H, J = 7.0 Hz), 1.13 (m), 2.15 (t, 4H, J = 7.0 Hz), 7.69 (s, 2H), 7.84 (d, 2H, J = 7.97), 7.93 (d, 2H, J = 7.97), 8.11 (s, 2H)
MALDI TOFMAS: m/z = 954.

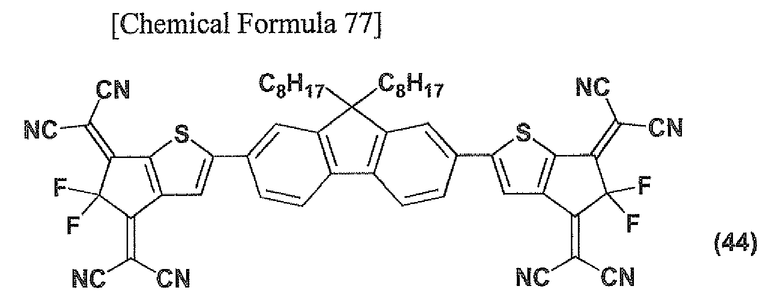

### (Example 7)

### <Synthesis of conjugated compound V>

After placing compound U represented by formula (45), compound K, Pd₂(dba)₃, tri-o-tolylphosphine and chlorobenzene in a 5 mL test tube, reaction was conducted under microwave irradiation (180°C, 5 min). Purification was performed with a silica gel column (chloroform) and GPC, to obtain conjugated compound V represented by formula (46).

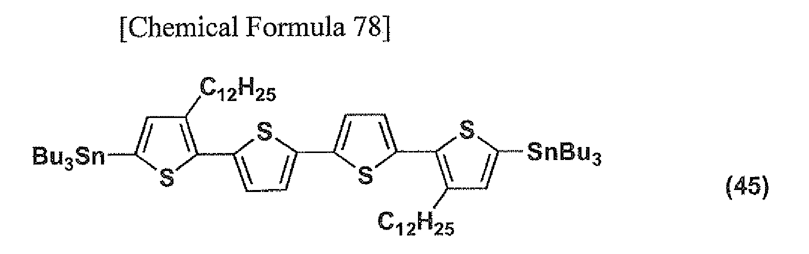

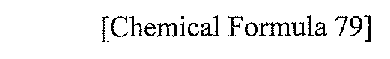

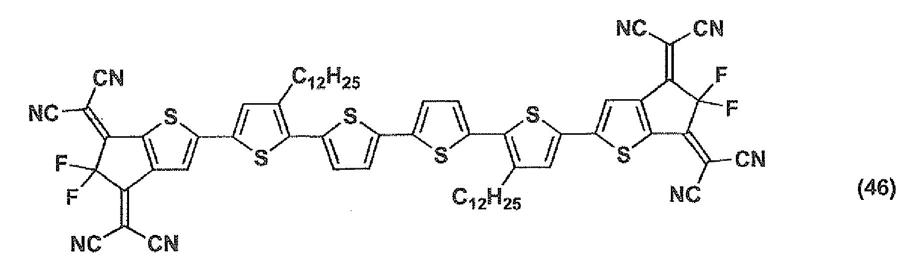

### (Synthesis of synthetic intermediate for conjugated compound 5)

### <Synthesis of Compound X>

Alter placing compound R (548 mg, 0.99 mmol), compound W represented by formula (47) (820 mg, 2.19 mmol), Pd (PPh₃)₄ (115 mg, 0.01 mmol) and toluene (10 mL) in a 20 mL test tube, reaction was conducted under microwave irradiation (180°C, 5 min). Purification was performed with a silica gel column (hexane) to obtain a liquid compound X represented by formula (48) (393 mg, 71% yield). The analysis results and chemical formula for the obtained compound X are shown below.
¹H NMR (400 MHz, CDCl₃) δ 0.79 (t, 6H, J = 6.9 Hz), 1.05 (m), 1.37 (m), 1.69 (m), 2.01 (m), 7.11 (m, 2H), 7.23 (m, 2H), 7.38 (m, 2H), 7.55 (m, 2H), 7.60 (m, 2H), 7.67 (m, 2H)

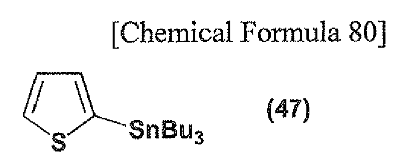

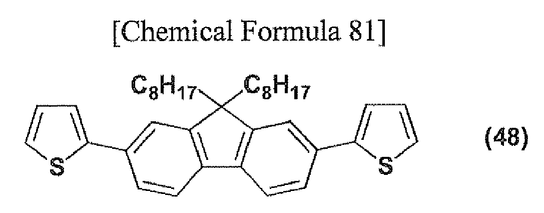

### <Synthesis of Compound Y>

Compound X (393 mg, 0.71 mmol) was placed in a 30 mL 2-necked flask, nitrogen substitution was performed, and THF (7 mL) was added. After cooling to -78°C, tetramethylethylenediamine (0.3 mL, 2.00 mmol) and n-BuLi (1.0 mL, 1.70 mmol) were added and reaction was conducted. After 1 hour, tributyltin chloride (0.5 mL, 1.70 mmol) was added at -78°C and the temperature was raised to room temperature. After another hour, water was added and extraction was performed with hexane. The organic layer was dried over sodium sulfate, the solvent was distilled off, and the residue was vacuum dried. Purification was performed with an alumina column (hexane) and GPC, to obtain compound Y represented by formula (49) as a yellow liquid (762 mg, 95% yield). The analysis results and chemical formula for the obtained compound Y are shown below.
¹H NMR (400 MHz, CDCl₃) δ 0.79 (m, 6H), 0.92 (m), 1.14 (m), 1.40 (m), 1.61 (m), 1.99 (m, 4H), 7.16 (d, 2H, J = 2.7 Hz), 7.50 (d, 2H, J = 2.7 Hz), 7.56 (m, 2H), 7.63 (m, 4H)

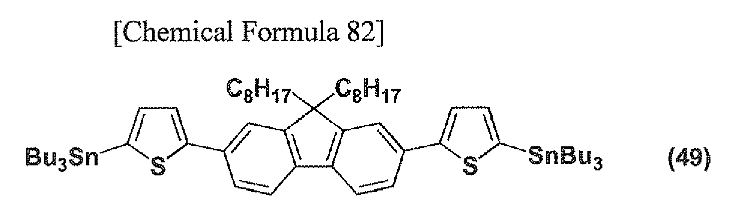

### (Example 8)

### <Synthesis of conjugated compound Z>

After placing compound M (155 mg, 0.42 mmol), compound Y (220 mg, 0.19 mmol), Pd₂(dba)₃ (3 mg, 0.003 mmol), tri-o-tolylphosphine (4 mg, 0.013 mmol) and chlorobenzene (3 mL) in a 5 mL test tube, reaction was conducted under microwave irradiation (180°C, 5 min). Purification was performed with a silica gel column (chloroform) and GPC, to obtain conjugated compound Z represented by formulas (50) as a blackish-green solid (59 mg, 45% yield).

The evaluation results for the obtained conjugated compound Z are as follows. Upon CV measurement of the obtained conjugated compound Z, a reversible reduction wave was observed at -0.66 V, and a low LUMO level and an excellent electron transport property were confirmed.
¹H NMR (400 MHz, CDCl₃) δ 0.67 (m), 0.78 (t, 6H, J = 6.9 Hz), 1.13 (m), 2.09 (m, 4H), 7.50 (d, 2H, J = 4.1 Hz), 7.61 (m, 2H), 7.65 (d, 2H, J = 4.1 Hz), 7.69 (m, 2H), 7.79 (m, 2H), 7.83 (s, 2H)

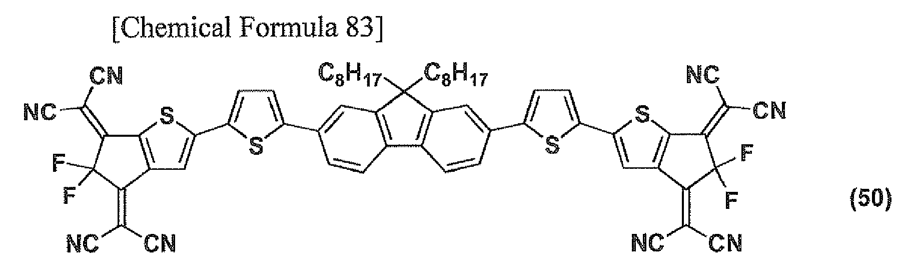

### (Comparative Examples 1 and 2)

Upon CV measurement of compound H as the synthetic intermediate for conjugated compound I (Comparative Example 1) and compound K as the synthetic intermediate for conjugated compound L (Comparative Example 2), reduction waves were observed at -1.85 V and -1.87 V, and it was confirmed that the LUMO levels were lower than with conjugated compound I and conjugated compound L, and that the electron transport properties were inadequate.

### (Example 9)

### <Fabrication of organic thin-film element 2 and evaluation of transistor property>

A silicon oxide film as the insulating layer was formed by thermal oxidation to a thickness of 300 nm on the surface of a highly doped p-type silicon substrate as the gate electrode. The lift-off method was used to form on this substrate a comb-shaped source electrode and drain electrode with a channel width of 38 mm and a channel length of 5 µm. The electrode-formed substrate was subjected to ultrasonic cleaning for 10 minutes in acetone and for 10 minutes in isopropyl alcohol, after which it was irradiated with ozone UV for 30 minutes to clean the surface. The cleaned substrate was dipped in hexamethyldisilazane (HMDS):chlorofbrm at room temperature and subjected to ultrasonic cleaning with chloroform to obtain an HMDS-surface-treated substrate. The conjugated compound N synthesized in Example 4 was used to prepare a 1 wt% solution of chloroform, as a coating solution. The coating solution of conjugated compound N was dropped onto the surface-treated substrate, and an organic thin-film of the conjugated compound N was formed by spin coating, to prepare an organic thin-film element 2. A Semiconductor Parameter Analyzer (trade name "4200-SCS", by Keithley Instruments, Inc.) was used to measure the organic transistor properties of the organic thin-film element 2 in a vacuum, while varying the gate voltage Vg in the range of 0-100 V and the source-drain voltage Vsd in the range of 0-100 V, and satisfactory n-type semiconductor Id-Vg characteristics were obtained. The mobility during this time was 1.5 × 10⁻⁴ cm²/Vs, the threshold voltage was 25 V and the on/off ratio was satisfactory at 10⁴. This confirmed that the organic thin-film element 2 employing conjugated compound N effectively functions as an n-type organic transistor, and that conjugated compound N can be utilized as an organic n-type semiconductor with an excellent electron transport property.

### (Example 10)

### <Fabrication of organic thin-film transistor 3 and evaluation of transistor property>

An organic thin-film element 3 having an organic thin-film of compound Q was fabricated, in the same manner as Example 9 except for using conjugated compound Q synthesized in Example 5 instead of conjugated compound N. The organic transistor property of the organic thin-film element 3 in a vacuum was measured in the same manner as Example 9, and a satisfactory n-type semiconductor Id-Vg characteristic was obtained. The mobility during this time was 7.5 × 10⁻⁵ cm²/Vs, the threshold voltage was 30 V and the on/off ratio was satisfactory at 10⁴. This confirmed that the organic thin-film element 2 employing conjugated compound Q effectively functions as an n-type organic transistor, and that conjugated compound Q can be utilized as an organic n-type semiconductor with an excellent electron transport property.

### Reference Signs List

1: Substrate, 2: organic semiconductor layer, 2a: organic semiconductor layer, 3: insulating layer, 4: gate electrode, 5: source electrode, 6: drain electrode, 7a: first electrode, 7b: second electrode, 8: charge generation layer, 100: first embodiment of organic thin-film transistor, 110: second embodiment of organic thin-film transistor, 120: third embodiment of organic thin-film transistor, 130: fourth embodiment of organic thin-film transistor, 140: fifth embodiment of organic thin-film transistor, 150: sixth embodiment of organic thin-film transistor, 160: seventh embodiment of organic thin-film transistor, 200: embodiment of solar cell, 300: first embodiment of optical sensor, 310: second embodiment of optical sensor, 320: third embodiment of optical sensor.

## Claims

1. A conjugated compound having a group represented by formula (I) and/or a group represented by formula (II). 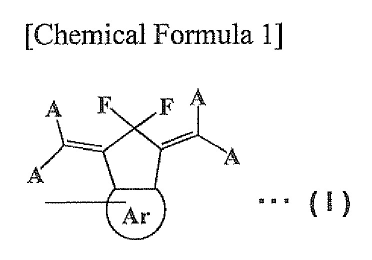 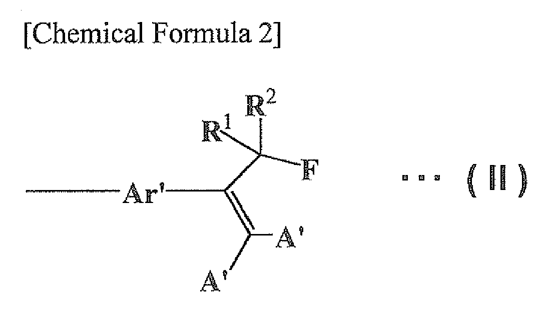 [In the formulas, Ar represents an optionally substituted trivalent aromatic hydrocarbon or optionally substituted trivalent heterocyclic group. A represents hydrogen, a halogen atom or a monovalent group, and when multiple A groups are present, they may be the same or different, and at least one A is an electron-withdrawing group. Ar' represents an optionally substituted C6 or greater divalent aromatic hydrocarbon or optionally substituted C4 or greater divalent heterocyclic group, and R¹ and R² are the same or different and each represents hydrogen, a halogen atom or a monovalent group. A' represents hydrogen, a halogen atom or a monovalent group, and when multiple A¹ groups are present, they may be the same or different, and at least one A' is an electron-withdrawing group.]

2. The conjugated compound according to claim 1, having a group represented by formula (III) as a group represented by formula (I). 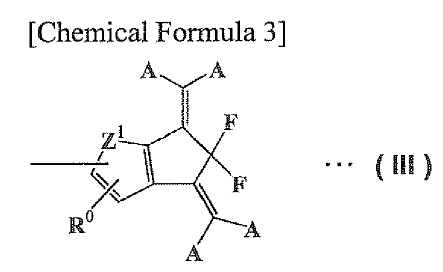 [In the formula, A represents hydrogen, a halogen atom or a monovalent group, and when multiple A groups are present, they may be the same or different, and at least one A is an electron-withdrawing group. R⁰ represents hydrogen or a C1-20 alkyl, C1-20 fluoroalkyl, C1-20 alkoxy or C1-20 fluoroalkoxy group. Z¹ represents a group represented by one of formulas (i)-(ix), wherein R³, R⁴, R⁵ and R⁶ are the same or different and each represents hydrogen or a monovalent group, and R³ and R⁴ may be bonded together to form a ring.]

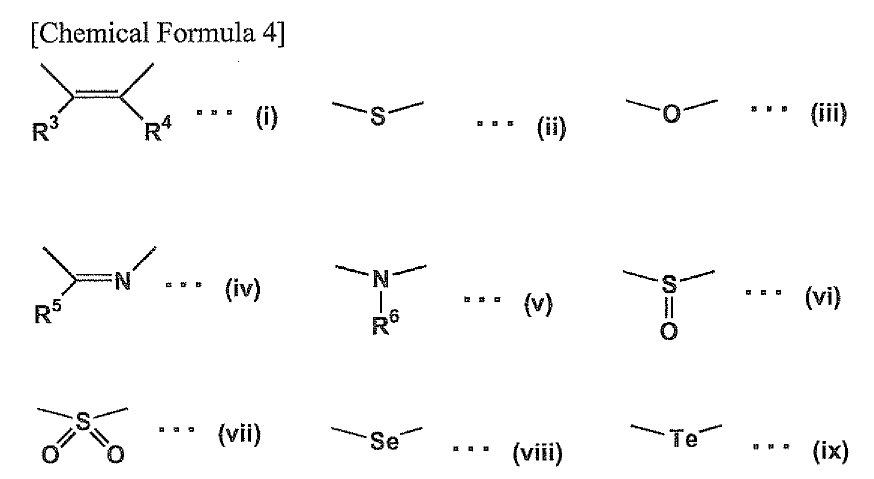

3. The conjugated compound according to claim 2, which comprises two or more groups represented by formula (III).

4. The conjugated compound according to claim 3, which is represented by formula (IV). 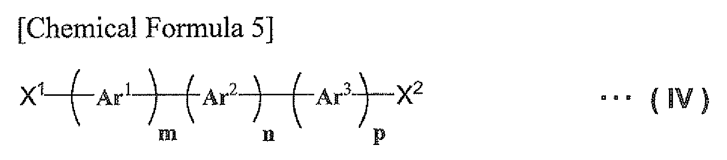 [In the formula, X¹ and X² are the same or different and each is a group represented by formula (III). Ar¹, Ar² and Ar³ are the same or different and each represents an optionally substituted C6 or greater divalent aromatic hydrocarbon or optionally substituted C4 or greater divalent heterocyclic group, and m, n and p are the same or different and each represents an integer of 0-6. This is with the proviso that m+n+p is an integer of 1 or greater.

5. The conjugated compound according to claim 4, wherein at least one of Ar¹, Ar² and Ar³ is an optionally substituted thienylene group.

6. The conjugated compound according to any one of claims 2 to 5, wherein Z¹ is a group represented by formula (ii).

7. The conjugated compound according to any one of claims 1 to 6, wherein A is a cyano group.

8. The conjugated compound according to any one of claims 1 to 7, having a group represented by formula (V) as a group represented by formula (II). 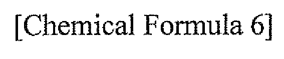 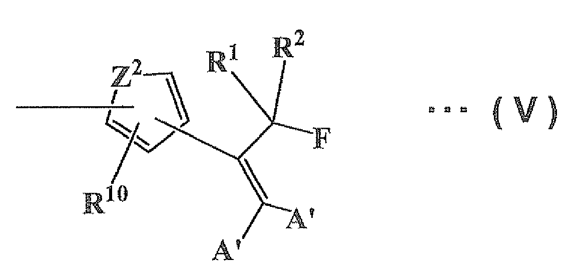 [In the formula, R¹ and R² are the same or different and each represents hydrogen, a halogen atom or a monovalent group. A' represents hydrogen, a halogen atom or a monovalent group, and when multiple A' groups are present, they may be the same or different, and at least one A' is an electron-withdrawing group. R¹⁰ represents hydrogen or a C1-20 alkyl, C1-20 fluoroalkyl, C1-20 alkoxy or C1-20 fluoroalkoxy group. Z² represents a group represented by one of formulas (xi)-(xix), wherein R¹³, R¹⁴, R¹⁵ and R¹⁶ are the same or different and each represents hydrogen or a monovalent group, and R¹³ and R¹⁴ may be bonded together to form a ring.]

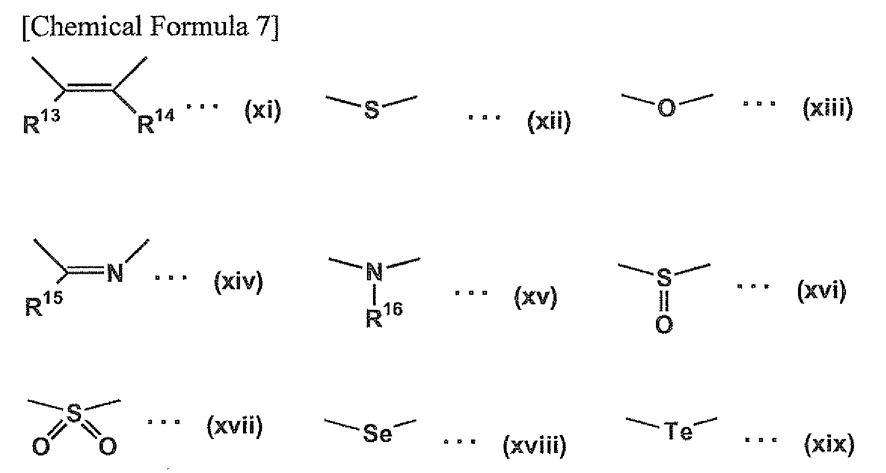

9. The conjugated compound according to claim 8, which comprises two or more groups represented by formula (V).

10. The conjugated compound according to claim 9, which is represented by formula (VI). 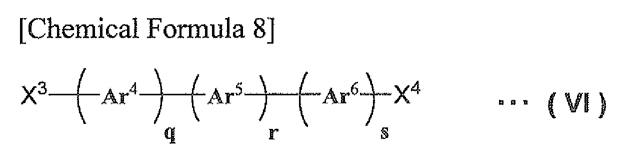 [In the formula, X³ and X⁴ are the same or different and is each a group represented by formula (V). Ar⁴, Ar⁵ and Ar⁶ are the same or different and each represents an optionally substituted C6 or greater divalent aromatic hydrocarbon or optionally substituted C4 or greater divalent heterocyclic group, and q, r and s are the same or different and each represents an integer of 0-6. This is with the proviso that q+r+s is an integer of 1 or greater.]

11. The conjugated compound according to claim 10, wherein at least one of Ar⁴, Ar⁵ and Ar⁶ is an optionally substituted thienylene group.

12. The conjugated compound according to any one of claims 8 to 11, wherein Z² is a group represented by formula (xi) or (xii).

13. The conjugated compound according to any one of claims 8 to 12, wherein A' is a cyano group.

14. An organic thin-film comprising the conjugated compound according to any one of claims 1 to 13.

15. An organic thin-flm element comprising the organic thin-film according to claim 14.

16. An organic thin-film transistor comprising a source electrode and drain electrode, an organic semiconductor layer serving as a current channel between the electrodes and a gate electrode that controls the level of current flowing through the current channel, wherein the organic semiconductor layer comprises the organic thin-film according to claim 14.

17. An organic solar cell comprising the organic thin-film according to claim 14.

18. An optical sensor comprising the organic thin-film according to claim 14.
